# EUROPEAN PATENT APPLICATION

(11) **EP 2 871 238 A1**
(43) Date of publication of application: **13.05.2015**
(21) Application number: 13306542.5
(22) Date of filing: 12.11.2013
(51) Int. Cl.: C12N 15/113, A61K 31/198, A61K 38/05

(54) **Agents modulating CoAA expression and/or activity for use in the treatment of diseases**

(71) Applicant: Centre National de la Recherche Scientifique (CNRS), 75016 Paris (FR); Université Montpellier 2 Sciences et Techniques, 34095 Montpellier Cedex 5 (FR)
(72) Inventor: Lecellier, Charles-Henri, 34920 Le Crès (FR); Vasselon, Thierry, 34090 Montpellier (FR); Bouttier, Manuella, Montreal, QC H2A 2C5 (CA)
(74) Representative: Blot, Philippe Robert Emile

(57) **Abstract**

The present invention relates to agents that modulate CoAA expression and/or activity and the use thereof for the treatment of diseases. Said agents are preferably selected from the group consisting of CoAA or CoAM expression inhibitors, RBM14 splicing modulators, agents which interfere or promote class I HDAC recruitment by CoAA and their combinations. Said agents are mainly intended for the treatment of viral diseases and/or cancer.

SEQUENCE LISTING

<110>  CENTRE NATIONAL DE LA RECHERCHE SCIENTIFIQUE
       UNIVERSITE MONTPELLIER 2, SCIENCES ET TECHNIQUES

<120>  AGENTS MODULATING CoAA EXPRESSION AND/OR ACTIVITY FOR USE IN THE
       TREATMENT OF DISEASES

<130>  BET 13P1267

<160>  31

<170>  PatentIn version 3.5

<210>  1
<211>  19
<212>  RNA
<213>  Artificial Sequence

<220>
<223>  CoAA siRNA

<400>  1
cccuugccuc cuaugguaa                                                    19


<210>  2
<211>  19
<212>  RNA
<213>  Artificial Sequence

<220>
<223>  CoAA siRNA

<400>  2
cgcuauucgg gcuccuaua                                                    19


<210>  3
<211>  23
<212>  DNA
<213>  Artificial Sequence

<220>
<223>  sense primer


<220>
<221>  misc_feature
<222>  (6)..(6)
<223>  n is a, c, g, or t

<220>
<221>  misc_feature
<222>  (15)..(15)
<223>  n is a, c, g, or t

<400>  3
ccagcncaca aaggnatagg agg                                               23


<210>  4
<211>  26

<212>  DNA
<213>  Artificial Sequence

<220>
<223>  antisense primer

<400>  4
cccctattcc tccccttctt ttaaaa                                          26


<210>  5
<211>  20
<212>  DNA
<213>  Artificial Sequence

<220>
<223>  sense, semi-nested primer

<400>  5
tggcaratgg attgyacaca                                                 20


<210>  6
<211>  19
<212>  RNA
<213>  Artificial Sequence

<220>
<223>  HDAC3 siRNA

<400>  6
ccaagagucu uaaugccuu                                                  19


<210>  7
<211>  19
<212>  RNA
<213>  Artificial Sequence

<220>
<223>  HDAC3 siRNA

<400>  7
ggcacccaau gaguucuau                                                  19


<210>  8
<211>  19
<212>  RNA
<213>  Artificial Sequence

<220>
<223>  HDAC3 siRNA

<400>  8
cauucaggau ggcauacaa                                                  19


<210>  9
<211>  19
<212>  RNA
<213>  Artificial Sequence

<220>
<223>  control siRNA

<400>  9
ccaugagcaa gauguccuu                                                    19


<210>  10
<211>  20
<212>  DNA
<213>  Artificial Sequence

<220>
<223>  CoAA sense primer

<400>  10
atcgagtgtg acgtggtgaa                                                   20


<210>  11
<211>  19
<212>  DNA
<213>  Artificial Sequence

<220>
<223>  CoAA antisense primer

<400>  11
cttctttgcc gttgagctg                                                    19


<210>  12
<211>  20
<212>  DNA
<213>  Artificial Sequence

<220>
<223>  CoAM sense primer

<400>  12
caaagaagtg aagggcaagc                                                   20


<210>  13
<211>  20
<212>  DNA
<213>  Artificial Sequence

<220>
<223>  CoAM antisense primer

<400>  13
tcagagagct cggctaaacg                                                   20


<210>  14
<211>  18
<212>  DNA
<213>  Artificial Sequence

<220>
<223>  Spliced HIV-1 RNAs sense primer

<400>  14
agtctctcaa gcggtggt                                                    18


<210>  15
<211>  24
<212>  DNA
<213>  Artificial Sequence

<220>
<223>  Spliced HIV-1 RNAs antisense primer

<400>  15
gactcatcaa gtttctctat caaa                                             24


<210>  16
<211>  18
<212>  DNA
<213>  Artificial Sequence

<220>
<223>  Unspliced HIV-1 RNAs sense primer

<400>  16
ctgaagcgcg cacggcaa                                                    18


<210>  17
<211>  20
<212>  DNA
<213>  Artificial Sequence

<220>
<223>  Unspliced HIV-1 RNAs antisense primer

<400>  17
gacgctctcg cacccatctc                                                  20


<210>  18
<211>  19
<212>  DNA
<213>  Artificial Sequence

<220>
<223>  HDAC3 sense primer

<400>  18
tgcattgtgc tccagtgtg                                                   19


<210>  19
<211>  22
<212>  DNA
<213>  Artificial Sequence

<220>
<223>  HDAC3 antisense primer

<400>  19
cttgacatat tcaacgcatt cc                                               22

<210>  20
<211>  18
<212>  DNA
<213>  Artificial Sequence

<220>
<223>  GAPDH sense primer

<400>  20
gaaggtgaag gtcggagt                                                    18


<210>  21
<211>  20
<212>  DNA
<213>  Artificial Sequence

<220>
<223>  GAPDH antisense primer

<400>  21
gaagatggtg atgggatttc                                                  20


<210>  22
<211>  20
<212>  DNA
<213>  Artificial Sequence

<220>
<223>  control sense primer

<400>  22
gtcgggttcc ttcaaactca                                                  20


<210>  23
<211>  18
<212>  DNA
<213>  Artificial Sequence

<220>
<223>  control antisense primer

<400>  23
gctcctgctg gcttcttg                                                    18


<210>  24
<211>  19
<212>  DNA
<213>  Artificial Sequence

<220>
<223>  HIV-1 LTR sense primer

<400>  24
cagagtcaca caacagacg                                                   19


<210>  25
<211>  19

<212>  DNA
<213>  Artificial Sequence

<220>
<223>  HIV-1 LTR antisense primer

<400>  25
taactaggga acccactgc                                                    19


<210>  26
<211>  44
<212>  DNA
<213>  Artificial Sequence

<220>
<223>  MS2 probe


<220>
<221>  misc_feature
<222>  (2)..(2)
<223>  n is amino-allyl-T

<220>
<221>  misc_feature
<222>  (15)..(15)
<223>  n is amino-allyl-T

<220>
<221>  misc_feature
<222>  (27)..(27)
<223>  n is amino-allyl-T

<220>
<221>  misc_feature
<222>  (43)..(43)
<223>  n is amino-allyl-T

<400>  26
anagtattcc cgggntcatt agatccnaag gtacctaatt gcna                        44


<210>  27
<211>  20353
<212>  DNA
<213>  Homo sapiens

<400>  27
tttctttttt tttttttttt ttttttgaga catgatctct tagtttgttg cccagcctgg      60

agcccagcgt catgattatc gtagctctgt gcaacctgaa actcctgggc acaagcaagc     120

tttttgcctc agcctcccag ctgggaccac tggtgcacat caccatgcct ggctaattgc     180

tctttttctt tttttttttt tgtttcagac agtttcactc tgtcacccag gctggagcac     240

agtggtatga tctcggctca ctgcaacctc cacctcccgg gttcaagcaa ttcttgtgcc     300

tcagcctctc aggtagctag gattatagac acgcgccagc actcccggct aattttttgt     360

atttttagta gagatggggt ttcaccatgt tgatcaggct ggtcttgaac tcctgacctc     420

aagtgataca cccacctcgg cctcccaaaa tgctgggatt acaggtgtga gccactgcgc 480

tgggactaat ttttatattt tttgagacat ggggtctccc tgggttgccc aggtttgtct 540

caaactcctg ggctcaagtg atcctcctgc cttggcctcc caaagtgctg ggattatagg 600

cattgagtca ctgcgtctgg ccaaacttct tactatgtgt cttacattgt gttcaacact 660

tgttattgct caaaatggcc ttatccttca tacatacctg ccaagagaaa tttttattag 720

agacaaggcc tccctgtgtt gctcaggctg gagtgcagtg gtgcgatcac agctcactgc 780

agccttagcc tcctgggctc aagtgatcct cccatctcag cctccagagt agctgggact 840

acaggcacac accaccacac ctggttaatt tttaaaattc ttttcatgta gatgggggtc 900

tatgtttctc aggctggtct tgaactcctg agctcaagtg atcctcctgc ctcagcctcc 960

caaagtgcta ggattacagg gatgagccac cacacccatc cccaaattta caattaaaaa 1020

actcttaaac ctaaaaacaa aaatgtggta aagccagtta ttttataggt aagatgatac 1080

tcatatgtgt aaagaatcta gctgggtgtg gtgggtcatg cctgtaatcc tagcacattg 1140

ggaggctgat tctggaggat cacttgaggc taggagtttg agagcagcca gggcaacaaa 1200

ttgacccccg tctctacaaa gtaaaaatta aaaaattatc tgggggtggt tgggcatggt 1260

ggttcatgcc tgtaatccta gcattttggg aggctgagct gggcagacca cgaggtcagg 1320

agttcaagac cagcccggaa ggtccaggct gcagggagcc atgatttcat cactgtactc 1380

cagcctgggg gacagagtga gactgtctcc aaaaaaccaa accaaaccaa aacaaacaaa 1440

caacaacaac aaagaaaaaa cctgcaaact tttctttgag atcagccaag ccaagctaga 1500

cactttacag atgagaaact cagtgagggc tgcaggagcg aagagggttg cccacctgga 1560

ccataaaact gaactagggc taatttaatg ctatttccat tattctagat caaggagtca 1620

gatccttatt caagccccag cattctgatt tgccagatct gtgaaatttg gcaagattct 1680

ttacatctat ttaattaatt tatgtattta gaaacagagt ttcactcttt tgcccaggtt 1740

agagtgcagt ggcaccatta cagctcacta taatcttaaa atcctgggct caagcgattc 1800

cttccccctc agtctctcaa gtagctagaa ctacaggtgc accaccaccc gcagatatat 1860

gtgtgtgtgt gtgtgtgtgt gtgtgtgtat atacatacac acacacacac acacacaaat 1920

atgtacgtat gtattttttg agacggagtc tcactctgtc acccaggctg gagtgcagcg 1980

gccacaatct cagctcaccg caacctccgc ctcctgggtt caagcaattc tcctgcctca 2040

gcctcctgag tagctgggat tacagacgca cgccactacg cccagataat tttttgtatg 2100

tttagtagag acggggtttc accgtgttgg ccaggctggt cccgaactcc tgacctcata 2160

atttgccttc ctcggcctcc caaagtgctg ggattacagg catgagccac cccgcccggc 2220

cgccggctaa tttaaaaaaa aaaaatttta gagatggatt ttggtcgttg cccaggctgt 2280

ccccaacctt ctgggctgag gcgatctacc tgcctcagcc tcccaaagca ctgggattac 2340

aggcatgagc caccacaccc agcctacaat tagtttcacc tgtttctttt caccttttaa 2400

aatgcagcta ctagaaaaat taaaagcacc tatgggtctc actgtgttaa cgtaagaccc 2460

cacctgccgg gtggggtgac tcacacctgt aatcccagca ctttgggagg ccgaggcagg 2520

cgaatcacaa ggtcgggagt tcgggaccag cctggccaac atgctgaaac ccctactaaa 2580

aatgcaaaaa ttacgccggg cgtggtggcg tgcgtgcctg taatcgcagc tactcgggag 2640

gctgaggcag ggaaaactgc ttgaacccag gaggcggagg ctgcagtgag ccgagatagc 2700

accactgcac tacagccagg gtgacaagag tgaaactctg tctcaataaa taaataaata 2760

aaatggctat ccttactggg tagctagggg tttgctgata tcctgttctg ggaatactgc 2820

cagcagtttg gatttcagga gataccagag tagtgcccct tttctttctt tctttctttc 2880

tttttttttt tttttttgag acggagtctc gctctgtcgc ccagcctgga gtgcagtggc 2940

gcgatctcgg ctcactgcaa gctccaactc ccgggttcac gccattctcc tgcctcagcc 3000

tccctagtag ctgggactac aggcccccgc caccatgccc ggctaatttt ttgtatttta 3060

gtagagacgg ggtttcaccg tgttagccag gatggtctcc atctcctgac ctcatgatcc 3120

gcccgcctca gcctcccaaa gtgctgggat tacaggcgtg agccacctcg cctggcccaa 3180

gcacatgcca tttcaatggc cttgtgctac acaactccca ccctcctatt ctgctctctg 3240

ttccaggttc tggccatatt gggctctttt tttttttttt ttttggagac agagtcctgc 3300

tctgttggcc aggatggagt gtggtggcgt gatctcagcc cactgcaacc tccacctcct 3360

gggttcaagt gattttaatg cctcagcctc ctgagtagct gggattacag gtgccagtca 3420

tcatgcatgg ctaatgtttt gttttcaatt agcaataatt gcgcctcgga taaacctcat 3480

tggctatgat actgccactg cgcaaagcta atgttttgta gttttagtag agacagggtt 3540

ttaccatgtt ggtcagactg gtctcgaact cttgacctca ggtgatccgc ccgcctcagc 3600

ctcccaaagt gctgggatta caggcgtgag ccaccgggcc tggcccatac tggctcttga 3660

ctcttccttg aaccagattt gcttctgccc cagcactctt tgacttgtac tttctggccg 3720

gcatgatctt ccctcagagg atggcttagt tcccttgatt actccaagtc ctcatcacca 3780

tctaacacgc ttatgtttta cttatttctt gcctgtttct ccccaccata agatccaaga 3840

aggttgggat ttctgtcatt tttttttgtt cactgctata ttcttggtgg tgtccacatg 3900

gtaggtgcta tttgttgact gaacacgtag gcaaatattt ccctgagttt gtcacatgtc 3960

tatgtttttg atgttgtctg ccatgcaaca atcttattta catactcaaa tctgtttaaa 4020

aaaatttttg agacggaatc tcactgtcgc ccatgctgga gtgcagtggc gtgatcttgg 4080

ctcactgcaa cctccgcctt ccgggttcaa gtgattctcc tgcctcagcc tcccaagtag 4140

ctgggattac aggcgcgcac caccacaccc ggctaatttt gtatttttag tagagacggg 4200

gtttcaccat tttggccagg ctggtcctga actcctgacc tccggtgatc tgcttgcctt 4260

ggtctcccaa agtgctggaa ttacaggcgt gagccaccgc tcccggacta aaattttttt 4320

taaggctcaa cttttgcttc ctgcagaatt aattttctcg tttattaaac tgtttattat 4380

gaaaaccttc aacatacaat aacgtcattc cattatttct tgactgggaa ttccatcagg 4440

gcagaggcct ttgatttttc accggctgcc aggacctcag caaccccagg gaatgtttcc 4500

aaagaatcaa ttaatggcac gagggcttct ccggggcctg cctcaggtcc cctcgcggtg 4560

ctcagcacag tcctgggagc cgtttgccga ctcaaggcgc ctgtcctggt ttctcccgcc 4620

aggcccggcc ttcccccgta cactcacgcg gcaaccccca ggcgcgggtt tggacagggc 4680

ttccagaatg ggaggagccc tgacttccaa ttgtacaaag ttttcctggg agcccgccgg 4740

tcccagcccc acccgggcca gagaccgcct tacagactag gcctctctgc cgccggcgcc 4800

gctcgcgcgc gttctcctct cttcagccct ccctcttctt cccaaccccc tctcccgcca 4860

cgggctgtgg cggttggtcg tccctttccc accccctcag ctaacgagcg ggctctaggc 4920

cctctgggat tggctgtgcc tgagcacatc ttccctcagc gtttctgatt ggtcgctgga 4980

cgcgcgggat gcgagtcccc attggctagg ccagggcgcc tgcgcggcgg ggttctcggt 5040

cgccagccat tcctgaggag gactgccggt cgttcggacg tcttgcctgt cgctggagga 5100

gaggtccggg ctctccagga aggtggctgc ggcgacaaaa tgaagatatt cgtgggcaac 5160

gtcgacgggg cggatacgac tccggaggag ctggcagccc tctttgcgcc ctacggcacg 5220

gtcatgagct gcgccgtcat gaaacagttc gccttcgtgc acatgcgcga gaacgcgggc 5280

gcgctgcgcg ccatcgaagc cctgcacggc cacgagctgc ggccggggcg cgcgctcgtg 5340

gtggagatgt cgcgcccaag gcctcttaat acttggaaga ttttcgtggg caatgtgtcg 5400

gctgcatgca cgagccagga actgcgcagc ctcttcgagc gccgcggacg cgtcatcgag 5460

tgtgacgtgg tgaaaggtaa cgcggaggcg cgctcggggg cgggggcgcg ctcggggcac 5520

tctgcctgtt agccacgccc cttacccggg ggtcggtcac tgcgcggttg ggaggggtgg 5580

ggaagtgcgc gggagcaggt cggaggtgtt gggggcgcgt tgggtagagc caccctcctc 5640

ccctgcggtc caggaaccgt ccaccaagta ggaggaagcg gctctgggtg ggtgcggcgg 5700

ccactgcgag ccaagctacg ggccggggga cgcgcctgag agccttgcga gtgttccggg 5760

ggcgcgcctt ctcctggtct cctgggcctg gcacccagcg gaaattggga agtgacgggc 5820

acaagccgga tcatgaagcg ggaagatttt ctccacttcc ccacttcctc tccagacgtc 5880

ggtcagagca agggaagagg gaaaaggtgg gggctgacgc cccaaggccg ccctcctgtt 5940

ccctcccgat gaatgtgctc aagcggaagg taacggggcc cttggacgtt tctcgggctg 6000

gggtctttta gtcttgtctg gcatgggtct actcatgggc acagttacac gtgggtacga 6060

gaggggcccc ttccgaatca ctttggcctt tcaatattag gtaggttagg cgtggcagct 6120

ctgtgctttt ctcctccctc aaacttgggg tttttctttg ggctcgattt tacatctgtc 6180

aactggaaac cccagccttt tgcatgtgca agaaagcttg ttttatgaag tccccttctg 6240

cgtagtcttt acatgccaag ccagtgccct agggcagtgt cattctttta taagcttggt 6300

gactttgcag gggggcatac tctggctgag ggggtcctgg ggaggggtct gtgagagctc 6360

ttcattaaac agtggctaaa cacggtttac tgaatcagta gtagtcattg cttgagtagt 6420

ctgattctca gggcagatcc cggtgccttt tcttcgagaa aaatgtgtca ccataattta 6480

agcctgacta gggacccagt tgactttttc atagcttgag tttttatccc ttaagctctt 6540

tatcttttac attggcaaga tttggtggga cagccgttgt gtatgtggcc agtctctgct 6600

ccacttggga acagatcaga gttaactttg ccctgtcatt ttggaagatg cagcctggga 6660

gtatctgatg ccctactcct ggtgggttca tacctgctca taccctgtgt gagaagtttc 6720

agggatagga aataaatgtg ggtgcaagtg cttatttatc ccaccaagat atttcatttg 6780

ctgtcctgga aaggcacatg tttggggcca ttcctagtcc tttaagtggg ggaaatataa 6840

agggtcatag tgtccactac taggaacaag tccttgttct gcattaatac tgcattctat 6900

tttcctcaac tcaggttata caagtccacg actatccgga atcgggcttt cctgctgggt 6960

gctctgaggc aggctgtatg gtgcatggat ttttgctttt gtttagagta aaagagtggt 7020

gggggagggt ctcagattgg gtacttgtcc agcaaaaggg tggggtatat gagtctcatt 7080

tgtgagagtt ttgtgtttaa ttgacttacc aagtttcttt tgtggttgtt aggtagagtg 7140

gctactaggt tgagagtcta gagtcaaaca tctgaaggga cagtctccct ggaactcagg 7200

cttcttgctg gctttcaggt ggtagagaaa gtcaaaaatt tgcctgaaat ttgcttggga 7260

cccaacacca agtgacgatg cgggtgaagt gcataactgc acatgaattt ttagaactcc 7320

tggctgactc cttgtgagag ttagacggag ttttgtgcat cttgttcctt tgtaaacatt 7380

tgattagcaa aaatgcattg acttactccg ggagtggtgg tcaactattt gcagttacat 7440

tgttgtgtaa cagagctcct gacatacttc gatgtaagag actgggaaag atttgaacat 7500

catatcctcc actgatgtct ttttatctgt ttgtgatgac ggaaagtgag ggttgaaagt 7560

tgggagtgta gtagaagcga ctggctctct aaagagcgta ctcatttctt atgctgggaa 7620

tggtcatttg attgagtctc cttgtgttgg ctgacagggc tttgaaaagc acagaagctt 7680

ggtgttgcac cagacaacca gctctgtttt atttcacagg aaaaaaaaac aacatagttg 7740

cttggttact ccaggagctt agataggatt tgtagaacag aaatctcaat agcaacagtg 7800

gattgtaacc actccacagc ttcctcctat tgggtgttca aagatgaaca gcttccggaa 7860

acatgtacag acttgtgggg agatctgaaa gacaaaagag gaaaatatct tctcttcatt 7920

tttgtgagtg actgtttctt cttcctatac agagctctgt agtttcaagt ttaagatttt 7980

ttctttttct tttttttttt ttttagacgg agtctggctc tgtcgcccag gctggagtgc 8040

actggcgcca tctcggctca ctgcaagctc cgcctcctgg gttcacgcca ttctcctgcc 8100

tcagcctccc gagtagctgg gactacaggc gcccgccacc acacccagct aattttttgt 8160

atttttagta gagacggggt ttcactgtgt tagccaggat ggtctcgatc tcctgacctt 8220

gtgatccgcc tgcctcagcc tcccaaagtg ctgggattac aggcgcaagc caccgcgcct 8280

agccctcaag tttaagattt ttagtcttaa gataggagaa cttgtttttc tggattcact 8340

taactttgtt actggaggac ttgtgatgcc accagaccat ttggagatct ctgtaaaaca 8400

ttacttgata gtcccaatgc agtgatttcc ttcagagttt ccttgaatta gatctgtggg 8460

caagaaggtt cctggaattt ttcagatctc tggttttaat ggtcttagga gtacttgggt 8520

gttagggcat ctttaatcta gctaggtctg tgctttgcac ttactgtgat ggggcttatc 8580

actacaggat gcagttgagt tagctgggca acctattcac acttttcccc tccagtgtca 8640

ggcttagaaa cttcctgata caatttggtt ataattgtat cagatggatt tttaaatttt 8700

gtatttattt ttatgttatt tatatattta ttattttttt ttatgagaca gagtctcatt 8760

ctgttgccca ggctggagtg cagtggtgag atcttggctc actgcaacct ccgtctcctg 8820

ggttcaagca attctcctgc ctcagcctct caagtagctg ggtctacagg ccaccacacc 8880

tggctaattt ttgaattttt agtagagaca gggtttcact atgttggcca ggctggtctt 8940

gaactcctga cctcaggtga tcttcctgcc tcggcctccc aaagtgttgg gattacaggt 9000

gtgagccacc atgcccggct tggattttta aatttttgga tcagttataa aactcatttg 9060

ggtagtcatt tgaatcatac atgtgaaatc tcaattttca gatttcacca cttggtctga 9120

ataaaactca ctaaggaggg aaacagacag tgggatgtcc aaatgctctt gtaagcccat 9180

ggaagttttt gtctcattct agtgtcagga agttgtggat agaataatca ggactgttag 9240

aattgttcct taatattccc tcagaatgtc tggaggcccc tccctgcaac aagtgcctat 9300

gagctccagc aactctgctt ccccttcact ttgggccttt ctgcttgcca tctccaggtg 9360

gttttgctat tcccgaggat tacctcagcc tctggtttta tttttatttt ttatttaaag 9420

attttttaaa atatttattc attctttagg gtctcgcttt gttgctcagg ccagagtgca 9480

gtggcgtgat tgtggctcac tgtagtcttg acctcctgag ctcaagccat ccttccacct 9540

cagtctccca aagtattagg attacaggag ggagccacca ttcctggccc agcctctgct 9600

tttaaagggc tcttaggcca catgctggtt ttttgtgttt tttttttaag cttactctat 9660

tcaaagtgga gatgaaggat cacaggacaa ctttagtgcc ttatgcctag gccaaaggaa 9720

acacgtagct tttcacaggt ggtagataag gctctggcca tgggagtgtt gaggacagag 9780

ttgtgataat tcattttgtt cttttttttt tttgagaggg agtttcatcc tttttgccca 9840

ggctggagta cagtggcacg atcttggctc actgcaacct ccgcctcccg ggttaaagtg 9900

attctcctgc ctcggcttct tgagtagctg ggattacagg cacatgccac cacagccggc 9960

taatttaata tttttagtaa agacggcgtt tcaccttgtt ggtcaggctg gtcttgaact 10020

cctgacctca ggtggtccgc ctgcctcggc ctccccaaag tgctgggatt atggtgtgag 10080

tcattgcgcc tggtctcatt gtgttctttt atcgtctgtg ccagcttcta agtttgcatg 10140

gcattgtgtt ttgcttgaga tgaaagataa tattctatag ggaaataaat ctctggttat 10200

ttctaggttt gaataatcat aattccttat tctgtagctg ttcatatctt acaaggcact 10260

tttgtctttt ctcttgaacc tcttaacttt gaggtaggta ggatagatgt gtcggaggaa 10320

atacatggta agtttctttt tatttttttt gagacggagt tttgcgcttg tcacccaggc 10380

tggagtgcag tgatcttggc tcactgcagc ctccttcttt ttcttccggg ttcaagtgat 10440

tctcctccct cagcctctgg agtagctggg attacaggtg ccagccacca cgccctggta 10500

atttttgtat ttttagtaga gacgtggttt caccatgttg gccaggctgg tctggaattc 10560

ctgacctcag gtgatccacc cacctgggct tcctaaagtg ttgggattac atgcgtgagc 10620

caccgtgctt ggctagtttc ttttcttttt ttcttttttt tttttttgga aatggagtct 10680

tgctgtgtca cccagactgg agtacggtgg tgccatttcg gctcactgca acttccacct 10740

cccaggttca agtgattctc ctgccccaga gcctcctgag tagctggaat tataggtgtg 10800

caccaccaca cctagctaat tttgtatttt ttagtagaga cagggtttca ccatgttggt 10860

caggctgatc ttgaactcct gatctcaggt gatctgcctg ccttggcctc ccaaagccct 10920

ggcatgagcc cctgtacctg cctaaatggt aagtttccta agatcttata acaagtcagt 10980

gatacctggg tctggaactg agaccattga atttctaatt aaggatccct tttgtgctat 11040

gccacacttc tgacttctac ttattgattg cattttttcc ccattatcct tagtcttgtg 11100

tcattgtctt caaatccagg gaaatgactt gttctgtagg agcagctaga taaggcatcc 11160

cagtacgaaa gtttctaaaa atgaggaccc ttgtttggag gttattaaga atgtttctta 11220

tcttagtatt gtcatctggg tagctctttg tttcttaagc tgatgtgaca gatagcatcc 11280

tggtacttgg atgctatcta agagtagcca caggtcgaga atggagctaa gcttagagcc 11340

ttatttccta ggggcaggat tctctccttc agattctgcc tgtgctttgt gagtgtggac 11400

atgagaattt ctttccattc cttcattccc tgaagttttc tccttctgtg tagtagataa 11460

gtatagcttt gagaccggca tcctgtcttc tcctaccaca gcttattcag gtcataatga 11520

gagctgcagg ggtaacctgg ttagcagagc ctgttagctg cagtaattgc agcttcatac 11580

cttataggtg agaaaccaga tctttctggt aagcagtctg ctctgtgaac agaagcccaa 11640

ggtgtaaggt atacctctgt cccttaaaga ggaaacagtg gaggatgcag tgtggtctgg 11700

gacaggggga ttgggcatga actcagccag tgggcttagt ttctattccc atacctcatt 11760

ggcttacaca gggtccatct tctctgcttc taagcctaaa tttttagtca tattacacag 11820

tcctgcctta taccttgact tctcagggat agattatgta gcaaaggggc attggaagag 11880

ctgtgattct ggtcctgact ctaacacact ttgagtgggg caagccatat aaaaactctt 11940

gagcatgtat gtccacctgt taaatggagg taatgattct ttgccctgcc tacctcacag 12000

gattgtgagg cccaaatgag gtcctcctgt acctaagagt gctttgaaaa cttgggaagt 12060

actgtgaaaa agcggagaga tcatattgga atgttgtgaa ctgcttgctt gttgcagtct 12120

atctctcttg ccttaattgg acatgggctg agagatgggt cataccaaaa cgtgagtggt 12180

catttccagt gtttgagagt taggcctcaa aagtgagcta ggcctcaaag gtgtttggtg 12240

agtttgggtt gagaacactg aagaactgag tgatctagga acgctttctg cattttgttc 12300

tgctggtttg tacttacagg agatgtgaag atttttatcc tgtgttgcag ttcatctttg 12360

ctccttcttc tctcaccagg aagttgggtg agatggcagg gtttgtaaag gggagaatgg 12420

gaagaaggct gtaggcaaag atgactgctt gggacagtca gaagctttgt tatatgggag 12480

ctacatttta tgacatactc ctggagagga gggtttggag gccttggagg tagctggcaa 12540

cagctgggtg ctgttgtgtg tccaatttgg gacccatcag gtagcatgcc ctgcccccct 12600

aattgctaac cctctgtctg ctgctttatc agactacgcg tttgttcaca tggagaagga 12660

agcagatgcc aaagccgcaa tcgcgcagct caacggcaaa gaagtgaagg gcaagcgcat 12720

caacgtggaa ctctccacca agggtcagaa gaaggggcct ggcctggctg tccagtctgg 12780

ggacaagacc aagaaaccag gggctgggga tacggccttc cctggaactg gtggcttctc 12840

tgccaccttc gactaccagc aggcttttgg caacagcact ggtggctttg atgggcaagc 12900

ccgtcagccc acaccaccct tctttggtcg cgaccgcagc cctctgcgcc gttcacctcc 12960

ccgagcctct tatgtggctc ctctgacggc ccagccagct acctaccggg cccagccgtc 13020

cgtgtcactg ggagctgcct acagggccca gccttctgcc tctttgggtg ttggctatcg 13080

gactcagccc atgacagccc aggcagcctc ttaccgcgct cagccctctg tctcccttgg 13140

ggcaccatac aggggccagc tggctagtcc tagctcccag tctgctgcag cttcttcact 13200

cggcccatat ggtggagccc agccctcagc ctcggccctt tcctcctatg ggggtcaggc 13260

agctgcagct tcttcgctca actcctatgg ggctcagggt tcctcccttg cctcctatgg 13320

taaccagcca tcctcttacg gcgcccaggc tgcctcttcc tatggggttc gtgcagctgc 13380

ttcttcctac aacacccagg gagcagcttc ctccttaggc tcctacgggg ctcaggcagc 13440

ctcctatggg gcccagtctg cagcctcctc actagcttat ggagcccagg cagcttcata 13500

taatgcccag ccctcggcct cttacaatgc ccagtctgcc ccatatgctg cacagcaggc 13560

tgcttcctac tcttcccaac ctgctgccta tgtggcacag ccagccacag ctgctgccta 13620

tgccagccag ccagcagcct acgccgcaca agccactacc ccaatggctg gctcctatgg 13680

ggcccagccg gttgtgcaga cccagctgaa tagttacggg gcccaagcat caatgggcct 13740

ttcaggctcc tatggggctc agtcggctgc tgcggccact ggctcctatg gtgccgcagc 13800

agcctacggg gcccaacctt ctgccaccct ggcagctcct taccgcactc agtcatcagc 13860

ctcattggct gcttcctatg ctgcccagca gcatccccag gctgctgcct cctaccgcgg 13920

ccagccaggc aatgcctacg atggggcagg tcagccgtct gcagcctacc tgtccatgtc 13980

ccagggggcc gttgccaacg ccaacagcac cccgccgccc tatgagcgta cccgcctctc 14040

cccaccccgg gccagctacg acgatcccta caaaaaggct gtcgccatgt cgaaaaggta 14100

ctgtatgccc ccccgcctct gcccccagct ggggcttagg gcaaggggct gaggttggca 14160

tgggagggaa actggaggca tcggcccctc cctcggtctt ctcttctcta tttttgtggg 14220

atgtccagag gccgagggga gcagtgtcta tgaactttcc tggtcaccag ggttcaggtg 14280

gagcatagtg ctcagcctgg ggtggtacct gctagtcagg ccaggcacag tgtcacctgt 14340

ctaggctagc ccaagtgtca ttggagctac tgcctgcagg actgtggccc atatcctttc 14400

ccactgtctc aaggatcctt aagcctatta cgtggttgtc ctggctttgg cagggatgtg 14460

ggttaaagag tagtcccttt ggcattcact ggctaaaggc aagtttacga ggtgcgtcca 14520

ctctgggaag ttggggacct cgctttcatc cgccgttctg ttgataaaca accaaggtct 14580

ttctctgcag ggcccagggg cggagatagt ttgctgtggt gtgggaatgt agaccaaaat 14640

ccaccttggc tagttcagag ggaggacttt cagtggaggg tttgatttcc cactgagttg 14700

aagccaggct tgattttttt ggcctggata gggtgatgag ccttctgaca aaaggagacc 14760

aatctcttgg agaccactgt gatcacaccc tccctgtccc cattggcatc tccccaatgc 14820

ccacctggag tcctcccctc aattgtctca ttcaccaact gtcttcttct ctcgactagg 14880

tatggttccg accggcgttt agccgagctc tctgattacc gccgtttatc agagtcgcag 14940

ctttcgttcc gccgctcgcc gacaaagtcc tcgctggatt accgtcgcct gcccgatgcc 15000

cattccgatt acgcacgcta ttcgggctcc tataatgatt acctgcgggc ggctcagatg 15060

cactctggct accagcgccg catgtagggc catcctggga tggggcacca cagggaggga 15120

gggagaaaag aggtgggtag ggttacagat ccaggttata actactctgg cccatacctt 15180

tcctggttgt ggtttttcat gccctctacc atgtgggcct tccccaggag atgatcctgt 15240

taagtgttcg gcagtaacct actttgttcc ttcgcctcag cagcaaatct tgctactggc 15300

tctagatctg cggtttcccc tctaccctgc ctcccgtctc cccagaatgg gaatttcttt 15360

tatgttttta tttttttcct ggctcccttt tatttttgtg cgcgatattt aaggtcgtct 15420

ggatggggaa gcaacctgca gctgaggtcg ccggcgcctt tttcttttta gatgggaagg 15480

aggccaggaa agggtcagct taaccatttc ctatgtgcca agctgtgcca gcagtccagg 15540

gtaccctgac tgtccctctg tagactgttg agactgagtt cctgttggga cagtcagttg 15600
gtatgtatcc aagtccctgc tgaccactaa tgttctagct gatggtgagc ggcacagtcc 15660
cacttcccca tctccccaag taggtggtgt tagaaaacct taattttttt tcccttttgt 15720
atggactaca aataaaactt ggggcaattt gcagtttgga aacctggttg tcattgtctt 15780
gattgcattc agtgtcaaag gagccaattt gacatccagg aagacatgat agctaaaggg 15840
aaggcagtca gagaacccag gactggtgag gaaaggggtt tgaatgtcgg aattagaagg 15900
attgccctgt ggcccagaag gatttagctc atcgtcgtcc tgctagcctg ccatctttca 15960
gactttccag ggcaggtggc ctggtcctca gccctcaact tgagttagtc acaccaaaat 16020
gcagtaaatg atgcccattt tcctctgcct gtgcttgacc attttcactg acttttctcc 16080
agttcaggaa taacctgtgt tccaatggga tggttctggg gtttggcctc ttaggaagtg 16140
gggaatgggc tgctgtggta tttacaggct agtagatcct ttaggcagta ggctggaatg 16200
agtgctgggg ctgggaccca gaaaggaacc agtagtggag tgggggtaat ggcgttgcct 16260
gatactgccc tatggttgga ataaggaagg gcagagaaaa cttgaagcag gtatggggca 16320
ttctggccat agagctcgta ttttgcctgt tgagctgtac catggagcat cctcctgtgt 16380
cctgccaacc cctgccatta tagtcaccgg agactccttt acctatccct atctgttagg 16440
gttttcagat gtcctctcgc tgggtttata tttgaaggta tagaatctgg gaaagggtgg 16500
ggtgcaagct aaccaaatag ggatgctagg gttgggtggg gaattgagga ctcttctgtg 16560
cttttattgt agggctgggg atcgaggatc tgggcaaaaa atatgacatt tccaaccagg 16620
gacaaaatgg aggcttgggc ttagtgatgg gtaaatggaa gactggttga gtgggatgat 16680
aatgggagaa cttactgatg ctcttttggg aaaaggtgct ttaaagactc gatttgggag 16740
cctactgggg caaggcgact ggggaggtgt acgtgttagt caagtggagg tcaggtgggt 16800
tatcttctgc ctccttcact ggcttcatgt gaacttgcct gtgacagaat atcttgccct 16860
agatgtcctc ttccctcttg ccatcgtgca aaagcattcg atcttatgac cgtgagttct 16920
tatttgtgga tgctaactgg ggtaatctca gggagtactg gggccatggc tctttccctt 16980
gcttctcttt ctgctcactg ctttgctttc aggtgcttag ctgctccttt ttatttcttt 17040
gcaggactgg gaaagcatgg gaggaggatt ttgataagtt tcttgttctc tgcgtggtga 17100
tggggtggtc tgagggccaa gttaataagc ctacctttgg ttattaccct ggaaaattga 17160
gggttgatgg tagggttatg attgtggcct gtgtatttgc aatttttctt tgtcagtgtg 17220
gctttctgtc atcttccttt ttctccagtg agggttctgc cactgtctca atcccttggt 17280
tcctgcaaaa aacagctaaa tgtgctctca gggccagtgg tgatgttctg tgttgcagcc 17340
ccagctcttg aaagtgacct ggcccagatg agctctgcct gcttgtcatc tttaggtcct 17400
gctgcccctg ctgcctcagc cctcaggctc ctaactgctc aagccagaag gagctacaaa 17460

gccaaatcat ttgtcatttt tctctggcag attgttttaa aatctccctc ccctctttta 17520

ttttaattat gccacctgtg ttaatcctat agcctgcttc tttgctttgt ggattactga 17580

tttcaagttc tataacttgg ggaaagttag gttgagtgaa tgggaggaca gacactgaat 17640

gtgtgttttg gaaaaatgga ctctctatct tcagattatt cagcttctcc acctatcacc 17700

tctacaactt ggccttcatt ggagaagttt tagttggctt attcctggaa caagaccacc 17760

tagaggcttt tagccttgtc agcctgccct cttctgaggt ttggaccttg ttttaaattg 17820

tgtgcatggt gggcttgaga ctctcttgta atggaggtaa tgggactatc actgaggggt 17880

tcagcctatc caggtaggct accgttctag tcatacccat cacttaaatt ctgccacagt 17940

ctgcacagac ggttggtcct ccaggcatat tgtcctatta agccaaatag gtggacatca 18000

tgggatggat gaggtcatca tgaaaacaga tttactggat ttatggacct agtatatact 18060

atgtaacaga tggaaaactg aattttacaa tctataggta cctttgaaag gtcataaagg 18120

gtaaaagtct atagttttcc agaaaataat tcttaaaact gggttttatg tctagattta 18180

aatcatgtat ctgtctgtag tgcttcaaat gtggccaggt gtggtagctc acacctgtaa 18240

ttcccagcgc tttgggaatt tgaggcaaga ggattgctag aggccaggat tttgagacca 18300

gcttcggcaa tacggtgaga ccttgtctct accaaaagaa aagaaaaaaa aaagccagct 18360

gtggtggcat gcacctgtag ttctagccac ttgggaggct gaagcaagag gattgcttga 18420

actcaggagt tggaggttat agtgaactat gatcatgcga ctgcacttca gcctgtatga 18480

cacagcaaga ccctgtctca aataaacaaa caaacaaatg ccttgctcag ctagctgagg 18540

caggctttgg gctgaccttg agggatttat tgtctgggag gcaggaccta cctcagtgag 18600

cgagtttata tgaataagta ttgataggca aatggtaggt aaggagggag aggatgtggc 18660

agtaatccca ggtagttaaa ttttttggag aaatgatttg atttaaaaac cgacttgggg 18720

caggtgtcat ggctcacgtt tgtaatccta gcactttggg aggccggggt ggttggatcc 18780

ttttgagtcc aggagttcaa gaccagcctg ggctacatgg gtaaacttca tctgtacaaa 18840

aaataaaaaa ctagccagcc gtggtggtac gtaccagtag tgcctgtagt ccagctgagg 18900

ctggaggatc tcttgagcct gggaggtgga gtttgcagtg agctaagatc atgccactgc 18960

actccagcct gggcaacaga gtaaaacccc gtctgaggaa aacataaaaa caacaacaaa 19020

aaagtaaacc gacttggtat agacaagagt gactgttgtc tctttaatca gtttaagttt 19080

tttttttttt tttaagaggg tctcactgtg ttgcctaggc tacacttgaa cacgtggctc 19140

aagtgatctt cctacctcag gctcccaagt agctgggact acaggaggtt tcctgaattt 19200

gaatcctggc tctccctctt cttggctgtg tgatcttgga catattagga acgctgcctc 19260

tattttgtta cctataaaat gaggataata gctaactaca gtaaattaaa tatattaata 19320

caacaaacta aatttatatt agtgtagcca acacaattat attaatcttc atagcaattc 19380

acgtgttagc tatttctttt tttttttttt ttaagatgga gtcttgctct tgtcacccag 19440

gcaggagtac agtggcgcaa tttcagctca ctgcaacctc tgcctcctgg gttcaagtga 19500

ttctcctgcc tcagcctccc tagtagatgg gattacaggc gcccgctatc acgcccagct 19560

aatttttgta tttttagtag agacaggatt tcaccatgtt ggccaggctg gtcttgaact 19620

cctgacctca ggtgatccac ttgcctcggc ctcccaaagt gctgggattg caggtgtgag 19680

ccaccatgcc ctgccaacat tagctatttc tttttttttt tgagtcggag tcttgctctg 19740

tcgcccaggc tggagtgcag tggtgcgatc ttggctcact acaagctccg cctcccgggt 19800

ttacgccatt cttctgcctc agcctcccga gtagctggga ctacaggtgc ctgccaccac 19860

ctcgcctggc taatttttgt atttttggta gagatggggt ttcaccgtgt tagccaggat 19920

ggtctcgatc tcctgacctc gtgatctgcc cgccttggcc tcccaaagtg cttggattac 19980

aggcgtgagc cactacgcct ggccaacatt agctatttct aaacctattt tggaaatatg 20040

ggagttgagg ctgttggtta agtgactatt cttttttttt tttttgatgg agtctcgtgt 20100

cacccaggga ggagtgcagt ggtgcgatct tggctcactg caacctcctg ggttcaagca 20160

attctgcctc agcctcctga gtagctggga ctacagggat gcaccactac acccagctaa 20220

tttttctatt tttagtagag atggggtttt accatattgg ccaggctggt ctcgaactcc 20280

tgacctcaaa tgatcctccc actcggcctc tcagagtgct gggattacgg gtgtgagcca 20340

ccatgcctgg cca 20353

<210> 28
<211> 5415
<212> DNA
<213> Homo sapiens

<220>
<221> CDS
<222> (140)..(2149)

<400> 28
attggctagg ccagggcgcc tgcgcggcgg ggttctcggt cgccagccat tcctgaggag 60

gactgccggt cgttcggacg tcttgcctgt cgctggagga gaggtccggg ctctccagga 120

aggtggctgc ggcgacaaa atg aag ata ttc gtg ggc aac gtc gac ggg gcg 172
Met Lys Ile Phe Val Gly Asn Val Asp Gly Ala
1               5                   10

gat acg act ccg gag gag ctg gca gcc ctc ttt gcg ccc tac ggc acg 220
Asp Thr Thr Pro Glu Glu Leu Ala Ala Leu Phe Ala Pro Tyr Gly Thr
            15                  20                  25

gtc atg agc tgc gcc gtc atg aaa cag ttc gcc ttc gtg cac atg cgc 268
Val Met Ser Cys Ala Val Met Lys Gln Phe Ala Phe Val His Met Arg
        30                  35                  40

gag aac gcg ggc gcg ctg cgc gcc atc gaa gcc ctg cac ggc cac gag 316
Glu Asn Ala Gly Ala Leu Arg Ala Ile Glu Ala Leu His Gly His Glu
    45                  50                  55

ctg cgg ccg ggg cgc gcg ctc gtg gtg gag atg tcg cgc cca agg cct 364
Leu Arg Pro Gly Arg Ala Leu Val Val Glu Met Ser Arg Pro Arg Pro
60                  65                  70                  75

ctt aat act tgg aag att ttc gtg ggc aat gtg tcg gct gca tgc acg 412
Leu Asn Thr Trp Lys Ile Phe Val Gly Asn Val Ser Ala Ala Cys Thr
                80                  85                  90

agc cag gaa ctg cgc agc ctc ttc gag cgc cgc gga cgc gtc atc gag 460
Ser Gln Glu Leu Arg Ser Leu Phe Glu Arg Arg Gly Arg Val Ile Glu
            95                  100                 105

tgt gac gtg gtg aaa gac tac gcg ttt gtt cac atg gag aag gaa gca 508
Cys Asp Val Val Lys Asp Tyr Ala Phe Val His Met Glu Lys Glu Ala
        110                 115                 120

gat gcc aaa gcc gca atc gcg cag ctc aac ggc aaa gaa gtg aag ggc 556
Asp Ala Lys Ala Ala Ile Ala Gln Leu Asn Gly Lys Glu Val Lys Gly
    125                 130                 135

aag cgc atc aac gtg gaa ctc tcc acc aag ggt cag aag aag ggg cct 604
Lys Arg Ile Asn Val Glu Leu Ser Thr Lys Gly Gln Lys Lys Gly Pro
140                 145                 150                 155

ggc ctg gct gtc cag tct ggg gac aag acc aag aaa cca ggg gct ggg 652
Gly Leu Ala Val Gln Ser Gly Asp Lys Thr Lys Lys Pro Gly Ala Gly
                160                 165                 170

gat acg gcc ttc cct gga act ggt ggc ttc tct gcc acc ttc gac tac 700
Asp Thr Ala Phe Pro Gly Thr Gly Gly Phe Ser Ala Thr Phe Asp Tyr
            175                 180                 185

cag cag gct ttt ggc aac agc act ggt ggc ttt gat ggg caa gcc cgt 748
Gln Gln Ala Phe Gly Asn Ser Thr Gly Gly Phe Asp Gly Gln Ala Arg
        190                 195                 200

cag ccc aca cca ccc ttc ttt ggt cgc gac cgc agc cct ctg cgc cgt 796
Gln Pro Thr Pro Pro Phe Phe Gly Arg Asp Arg Ser Pro Leu Arg Arg
    205                 210                 215

tca cct ccc cga gcc tct tat gtg gct cct ctg acg gcc cag cca gct 844
Ser Pro Pro Arg Ala Ser Tyr Val Ala Pro Leu Thr Ala Gln Pro Ala
220                 225                 230                 235

acc tac cgg gcc cag ccg tcc gtg tca ctg gga gct gcc tac agg gcc 892
Thr Tyr Arg Ala Gln Pro Ser Val Ser Leu Gly Ala Ala Tyr Arg Ala
                240                 245                 250

cag cct tct gcc tct ttg ggt gtt ggc tat cgg act cag ccc atg aca 940
Gln Pro Ser Ala Ser Leu Gly Val Gly Tyr Arg Thr Gln Pro Met Thr
            255                 260                 265

gcc cag gca gcc tct tac cgc gct cag ccc tct gtc tcc ctt ggg gca 988
Ala Gln Ala Ala Ser Tyr Arg Ala Gln Pro Ser Val Ser Leu Gly Ala
        270                 275                 280

cca tac agg ggc cag ctg gct agt cct agc tcc cag tct gct gca gct 1036
Pro Tyr Arg Gly Gln Leu Ala Ser Pro Ser Ser Gln Ser Ala Ala Ala

285                 290                 295

tct tca ctc ggc cca tat ggt gga gcc cag ccc tca gcc tcg gcc ctt        1084
Ser Ser Leu Gly Pro Tyr Gly Gly Ala Gln Pro Ser Ala Ser Ala Leu
300                 305                 310                 315

tcc tcc tat ggg ggt cag gca gct gca gct tct tcg ctc aac tcc tat        1132
Ser Ser Tyr Gly Gly Gln Ala Ala Ala Ala Ser Ser Leu Asn Ser Tyr
                320                 325                 330

ggg gct cag ggt tcc tcc ctt gcc tcc tat ggt aac cag cca tcc tct        1180
Gly Ala Gln Gly Ser Ser Leu Ala Ser Tyr Gly Asn Gln Pro Ser Ser
            335                 340                 345

tac ggc gcc cag gct gcc tct tcc tat ggg gtt cgt gca gct gct tct        1228
Tyr Gly Ala Gln Ala Ala Ser Ser Tyr Gly Val Arg Ala Ala Ala Ser
        350                 355                 360

tcc tac aac acc cag gga gca gct tcc tcc tta ggc tcc tac ggg gct        1276
Ser Tyr Asn Thr Gln Gly Ala Ala Ser Ser Leu Gly Ser Tyr Gly Ala
    365                 370                 375

cag gca gcc tcc tat ggg gcc cag tct gca gcc tcc tca cta gct tat        1324
Gln Ala Ala Ser Tyr Gly Ala Gln Ser Ala Ala Ser Ser Leu Ala Tyr
380                 385                 390                 395

gga gcc cag gca gct tca tat aat gcc cag ccc tcg gcc tct tac aat        1372
Gly Ala Gln Ala Ala Ser Tyr Asn Ala Gln Pro Ser Ala Ser Tyr Asn
                400                 405                 410

gcc cag tct gcc cca tat gct gca cag cag gct gct tcc tac tct tcc        1420
Ala Gln Ser Ala Pro Tyr Ala Ala Gln Gln Ala Ala Ser Tyr Ser Ser
            415                 420                 425

caa cct gct gcc tat gtg gca cag cca gcc aca gct gct gcc tat gcc        1468
Gln Pro Ala Ala Tyr Val Ala Gln Pro Ala Thr Ala Ala Ala Tyr Ala
        430                 435                 440

agc cag cca gca gcc tac gcc gca caa gcc act acc cca atg gct ggc        1516
Ser Gln Pro Ala Ala Tyr Ala Ala Gln Ala Thr Thr Pro Met Ala Gly
    445                 450                 455

tcc tat ggg gcc cag ccg gtt gtg cag acc cag ctg aat agt tac ggg        1564
Ser Tyr Gly Ala Gln Pro Val Val Gln Thr Gln Leu Asn Ser Tyr Gly
460                 465                 470                 475

gcc caa gca tca atg ggc ctt tca ggc tcc tat ggg gct cag tcg gct        1612
Ala Gln Ala Ser Met Gly Leu Ser Gly Ser Tyr Gly Ala Gln Ser Ala
                480                 485                 490

gct gcg gcc act ggc tcc tat ggt gcc gca gca gcc tac ggg gcc caa        1660
Ala Ala Ala Thr Gly Ser Tyr Gly Ala Ala Ala Ala Tyr Gly Ala Gln
            495                 500                 505

cct tct gcc acc ctg gca gct cct tac cgc act cag tca tca gcc tca        1708
Pro Ser Ala Thr Leu Ala Ala Pro Tyr Arg Thr Gln Ser Ser Ala Ser
        510                 515                 520

ttg gct gct tcc tat gct gcc cag cag cat ccc cag gct gct gcc tcc        1756
Leu Ala Ala Ser Tyr Ala Ala Gln Gln His Pro Gln Ala Ala Ala Ser
    525                 530                 535

tac cgc ggc cag cca ggc aat gcc tac gat ggg gca ggt cag ccg tct        1804

Tyr Arg Gly Gln Pro Gly Asn Ala Tyr Asp Gly Ala Gly Gln Pro Ser
540                 545                 550                 555

gca gcc tac ctg tcc atg tcc cag ggg gcc gtt gcc aac gcc aac agc      1852
Ala Ala Tyr Leu Ser Met Ser Gln Gly Ala Val Ala Asn Ala Asn Ser
                560                 565                 570

acc ccg ccg ccc tat gag cgt acc cgc ctc tcc cca ccc cgg gcc agc      1900
Thr Pro Pro Pro Tyr Glu Arg Thr Arg Leu Ser Pro Pro Arg Ala Ser
            575                 580                 585

tac gac gat ccc tac aaa aag gct gtc gcc atg tcg aaa agg tat ggt      1948
Tyr Asp Asp Pro Tyr Lys Lys Ala Val Ala Met Ser Lys Arg Tyr Gly
        590                 595                 600

tcc gac cgg cgt tta gcc gag ctc tct gat tac cgc cgt tta tca gag      1996
Ser Asp Arg Arg Leu Ala Glu Leu Ser Asp Tyr Arg Arg Leu Ser Glu
    605                 610                 615

tcg cag ctt tcg ttc cgc cgc tcg ccg aca aag tcc tcg ctg gat tac      2044
Ser Gln Leu Ser Phe Arg Arg Ser Pro Thr Lys Ser Ser Leu Asp Tyr
620                 625                 630                 635

cgt cgc ctg ccc gat gcc cat tcc gat tac gca cgc tat tcg ggc tcc      2092
Arg Arg Leu Pro Asp Ala His Ser Asp Tyr Ala Arg Tyr Ser Gly Ser
                640                 645                 650

tat aat gat tac ctg cgg gcg gct cag atg cac tct ggc tac cag cgc      2140
Tyr Asn Asp Tyr Leu Arg Ala Ala Gln Met His Ser Gly Tyr Gln Arg
            655                 660                 665

cgc atg tag ggccatcctg ggatggggca ccacagggag ggagggagaa              2189
Arg Met

aagaggtggg tagggttaca gatccaggtt ataactactc tggcccatac ctttcctggt      2249

tgtggttttt catgccctct accatgtggg ccttccccag gagatgatcc tgttaagtgt      2309

tcggcagtaa cctactttgt tccttcgcct cagcagcaaa tcttgctact ggctctagat      2369

ctgcggtttc ccctctaccc tgcctcccgt ctccccagaa tgggaatttc ttttatgttt      2429

ttattttttt cctggctccc ttttattttt gtgcgcgata tttaaggtcg tctggatggg      2489

gaagcaacct gcagctgagg tcgccggcgc ctttttcttt ttagatggga aggaggccag      2549

gaaagggtca gcttaaccat ttcctatgtg ccaagctgtg ccagcagtcc agggtaccct      2609

gactgtccct ctgtagactg ttgagactga gttcctgttg ggacagtcag ttggtatgta      2669

tccaagtccc tgctgaccac taatgttcta gctgatggtg agcggcacag tcccacttcc      2729

ccatctcccc aagtaggtgg tgttagaaaa ccttaatttt ttttcccttt tgtatggact      2789

acaaataaaa cttggggcaa tttgcagttt ggaaacctgg ttgtcattgt cttgattgca      2849

ttcagtgtca aaggagccaa tttgacatcc aggaagacat gatagctaaa gggaaggcag      2909

tcagagaacc caggactggt gaggaaaggg gtttgaatgt cggaattaga aggattgccc      2969

tgtggcccag aaggatttag ctcatcgtcg tcctgctagc ctgccatctt tcagactttc      3029

cagggcaggt ggcctggtcc tcagccctca acttgagtta gtcacaccaa aatgcagtaa 3089

atgatgccca ttttcctctg cctgtgcttg accattttca ctgacttttc tccagttcag 3149

gaataacctg tgttccaatg ggatggttct ggggtttggc ctcttaggaa gtggggaatg 3209

ggctgctgtg gtatttacag gctagtagat cctttaggca gtaggctgga atgagtgctg 3269

gggctgggac ccagaaagga accagtagtg gagtgggggt aatggcgttg cctgatactg 3329

ccctatggtt ggaataagga agggcagaga aaacttgaag caggtatggg gcattctggc 3389

catagagctc gtattttgcc tgttgagctg taccatggag catcctcctg tgtcctgcca 3449

acccctgcca ttatagtcac cggagactcc tttacctatc cctatctgtt agggttttca 3509

gatgtcctct cgctgggttt atatttgaag gtatagaatc tgggaaaggg tggggtgcaa 3569

gctaaccaaa tagggatgct agggttgggt ggggaattga ggactcttct gtgcttttat 3629

tgtagggctg gggatcgagg atctgggcaa aaaatatgac atttccaacc agggacaaaa 3689

tggaggcttg ggcttagtga tgggtaaatg gaagactggt tgagtgggat gataatggga 3749

gaacttactg atgctctttt gggaaaaggt gctttaaaga ctcgatttgg gagcctactg 3809

gggcaaggcg actggggagg tgtacgtgtt agtcaagtgg aggtcaggtg ggttatcttc 3869

tgcctccttc actggcttca tgtgaacttg cctgtgacag aatatcttgc cctagatgtc 3929

ctcttccctc ttgccatcgt gcaaaagcat tcgatcttat gaccgtgagt tcttatttgt 3989

ggatgctaac tggggtaatc tcagggagta ctggggccat ggctctttcc cttgcttctc 4049

tttctgctca ctgctttgct ttcaggtgct tagctgctcc tttttatttc tttgcaggac 4109

tgggaaagca tgggaggagg attttgataa gtttcttgtt ctctgcgtgg tgatggggtg 4169

gtctgagggc caagttaata agcctacctt tggttattac cctggaaaat tgagggttga 4229

tggtagggtt atgattgtgg cctgtgtatt tgcaattttt ctttgtcagt gtggctttct 4289

gtcatcttcc tttttctcca gtgagggttc tgccactgtc tcaatccctt ggttcctgca 4349

aaaaacagct aaatgtgctc tcagggccag tggtgatgtt ctgtgttgca gccccagctc 4409

ttgaaagtga cctggcccag atgagctctg cctgcttgtc atctttaggt cctgctgccc 4469

ctgctgcctc agccctcagg ctcctaactg ctcaagccag aaggagctac aaagccaaat 4529

catttgtcat ttttctctgg cagattgttt taaaatctcc ctcccctctt ttattttaat 4589

tatgccacct gtgttaatcc tatagcctgc ttctttgctt tgtggattac tgatttcaag 4649

ttctataact tggggaaagt taggttgagt gaatgggagg acagacactg aatgtgtgtt 4709

ttggaaaaat ggactctcta tcttcagatt attcagcttc tccacctatc acctctacaa 4769

cttggccttc attggagaag ttttagttgg cttattcctg gaacaagacc acctagaggc 4829

ttttagcctt gtcagcctgc cctcttctga ggtttggacc ttgttttaaa ttgtgtgcat 4889

ggtgggcttg agactctctt gtaatggagg taatgggact atcactgagg ggttcagcct 4949

atccaggtag gctaccgttc tagtcatacc catcacttaa attctgccac agtctgcaca 5009

gacggttggt cctccaggca tattgtccta ttaagccaaa taggtggaca tcatgggatg 5069

gatgaggtca tcatgaaaac agatttactg gatttatgga cctagtatat actatgtaac 5129

agatggaaaa ctgaatttta caatctatag gtacctttga aaggtcataa agggtaaaag 5189

tctatagttt tccagaaaat aattcttaaa actgggtttt atgtctagat ttaaatcatg 5249

tatctgtctg tagtgcttca aatgtggcca ggtgtggtag ctcacacctg taattcccag 5309

cgctttggga atttgaggca agaggattgc tagaggccag gattttgaga ccagcttcgg 5369

caatacggtg agaccttgtc tctaccaaaa gaaaagaaaa aaaaaa 5415

<210> 29
<211> 669
<212> PRT
<213> Homo sapiens

<400> 29

Met Lys Ile Phe Val Gly Asn Val Asp Gly Ala Asp Thr Thr Pro Glu
1               5                   10                  15

Glu Leu Ala Ala Leu Phe Ala Pro Tyr Gly Thr Val Met Ser Cys Ala
            20                  25                  30

Val Met Lys Gln Phe Ala Phe Val His Met Arg Glu Asn Ala Gly Ala
        35                  40                  45

Leu Arg Ala Ile Glu Ala Leu His Gly His Glu Leu Arg Pro Gly Arg
    50                  55                  60

Ala Leu Val Val Glu Met Ser Arg Pro Arg Pro Leu Asn Thr Trp Lys
65                  70                  75                  80

Ile Phe Val Gly Asn Val Ser Ala Ala Cys Thr Ser Gln Glu Leu Arg
                85                  90                  95

Ser Leu Phe Glu Arg Arg Gly Arg Val Ile Glu Cys Asp Val Val Lys
            100                 105                 110

Asp Tyr Ala Phe Val His Met Glu Lys Glu Ala Asp Ala Lys Ala Ala
        115                 120                 125

Ile Ala Gln Leu Asn Gly Lys Glu Val Lys Gly Lys Arg Ile Asn Val
    130                 135                 140

Glu Leu Ser Thr Lys Gly Gln Lys Lys Gly Pro Gly Leu Ala Val Gln
145                 150                 155                 160

Ser Gly Asp Lys Thr Lys Lys Pro Gly Ala Gly Asp Thr Ala Phe Pro
                165                 170                 175

Gly Thr Gly Gly Phe Ser Ala Thr Phe Asp Tyr Gln Gln Ala Phe Gly
            180                 185                 190

Asn Ser Thr Gly Gly Phe Asp Gly Gln Ala Arg Gln Pro Thr Pro Pro
        195                 200                 205

Phe Phe Gly Arg Asp Arg Ser Pro Leu Arg Arg Ser Pro Pro Arg Ala
    210                 215                 220

Ser Tyr Val Ala Pro Leu Thr Ala Gln Pro Ala Thr Tyr Arg Ala Gln
225                 230                 235                 240

Pro Ser Val Ser Leu Gly Ala Ala Tyr Arg Ala Gln Pro Ser Ala Ser
                245                 250                 255

Leu Gly Val Gly Tyr Arg Thr Gln Pro Met Thr Ala Gln Ala Ala Ser
            260                 265                 270

Tyr Arg Ala Gln Pro Ser Val Ser Leu Gly Ala Pro Tyr Arg Gly Gln
        275                 280                 285

Leu Ala Ser Pro Ser Ser Gln Ser Ala Ala Ala Ser Ser Leu Gly Pro
    290                 295                 300

Tyr Gly Gly Ala Gln Pro Ser Ala Ser Ala Leu Ser Ser Tyr Gly Gly
305                 310                 315                 320

Gln Ala Ala Ala Ala Ser Ser Leu Asn Ser Tyr Gly Ala Gln Gly Ser
                325                 330                 335

Ser Leu Ala Ser Tyr Gly Asn Gln Pro Ser Ser Tyr Gly Ala Gln Ala
            340                 345                 350

Ala Ser Ser Tyr Gly Val Arg Ala Ala Ala Ser Ser Tyr Asn Thr Gln
        355                 360                 365

Gly Ala Ala Ser Ser Leu Gly Ser Tyr Gly Ala Gln Ala Ala Ser Tyr
    370                 375                 380

Gly Ala Gln Ser Ala Ala Ser Ser Leu Ala Tyr Gly Ala Gln Ala Ala
385                 390                 395                 400

Ser Tyr Asn Ala Gln Pro Ser Ala Ser Tyr Asn Ala Gln Ser Ala Pro

                405                 410                 415

Tyr Ala Ala Gln Gln Ala Ala Ser Tyr Ser Ser Gln Pro Ala Ala Tyr
            420                 425                 430

Val Ala Gln Pro Ala Thr Ala Ala Ala Tyr Ala Ser Gln Pro Ala Ala
        435                 440                 445

Tyr Ala Ala Gln Ala Thr Thr Pro Met Ala Gly Ser Tyr Gly Ala Gln
    450                 455                 460

Pro Val Val Gln Thr Gln Leu Asn Ser Tyr Gly Ala Gln Ala Ser Met
465                 470                 475                 480

Gly Leu Ser Gly Ser Tyr Gly Ala Gln Ser Ala Ala Ala Ala Thr Gly
                485                 490                 495

Ser Tyr Gly Ala Ala Ala Ala Tyr Gly Ala Gln Pro Ser Ala Thr Leu
            500                 505                 510

Ala Ala Pro Tyr Arg Thr Gln Ser Ser Ala Ser Leu Ala Ala Ser Tyr
        515                 520                 525

Ala Ala Gln Gln His Pro Gln Ala Ala Ala Ser Tyr Arg Gly Gln Pro
    530                 535                 540

Gly Asn Ala Tyr Asp Gly Ala Gly Gln Pro Ser Ala Ala Tyr Leu Ser
545                 550                 555                 560

Met Ser Gln Gly Ala Val Ala Asn Ala Asn Ser Thr Pro Pro Pro Tyr
                565                 570                 575

Glu Arg Thr Arg Leu Ser Pro Pro Arg Ala Ser Tyr Asp Asp Pro Tyr
            580                 585                 590

Lys Lys Ala Val Ala Met Ser Lys Arg Tyr Gly Ser Asp Arg Arg Leu
        595                 600                 605

Ala Glu Leu Ser Asp Tyr Arg Arg Leu Ser Glu Ser Gln Leu Ser Phe
    610                 615                 620

Arg Arg Ser Pro Thr Lys Ser Ser Leu Asp Tyr Arg Arg Leu Pro Asp
625                 630                 635                 640

Ala His Ser Asp Tyr Ala Arg Tyr Ser Gly Ser Tyr Asn Asp Tyr Leu
                645                 650                 655

Arg Ala Ala Gln Met His Ser Gly Tyr Gln Arg Arg Met
            660                 665

<210>  30
<211>  4061
<212>  DNA
<213>  Homo sapiens

<220>
<221>  CDS
<222>  (140)..(610)

<400>  30
attggctagg ccagggcgcc tgcgcggcgg ggttctcggt cgccagccat tcctgaggag      60

gactgccggt cgttcggacg tcttgcctgt cgctggagga gaggtccggg ctctccagga     120

aggtggctgc ggcgacaaa atg aag ata ttc gtg ggc aac gtc gac ggg gcg      172
                     Met Lys Ile Phe Val Gly Asn Val Asp Gly Ala
                     1               5                   10

gat acg act ccg gag gag ctg gca gcc ctc ttt gcg ccc tac ggc acg      220
Asp Thr Thr Pro Glu Glu Leu Ala Ala Leu Phe Ala Pro Tyr Gly Thr
            15                  20                  25

gtc atg agc tgc gcc gtc atg aaa cag ttc gcc ttc gtg cac atg cgc      268
Val Met Ser Cys Ala Val Met Lys Gln Phe Ala Phe Val His Met Arg
        30                  35                  40

gag aac gcg ggc gcg ctg cgc gcc atc gaa gcc ctg cac ggc cac gag      316
Glu Asn Ala Gly Ala Leu Arg Ala Ile Glu Ala Leu His Gly His Glu
    45                  50                  55

ctg cgg ccg ggg cgc gcg ctc gtg gtg gag atg tcg cgc cca agg cct      364
Leu Arg Pro Gly Arg Ala Leu Val Val Glu Met Ser Arg Pro Arg Pro
60                  65                  70                  75

ctt aat act tgg aag att ttc gtg ggc aat gtg tcg gct gca tgc acg      412
Leu Asn Thr Trp Lys Ile Phe Val Gly Asn Val Ser Ala Ala Cys Thr
                80                  85                  90

agc cag gaa ctg cgc agc ctc ttc gag cgc cgc gga cgc gtc atc gag      460
Ser Gln Glu Leu Arg Ser Leu Phe Glu Arg Arg Gly Arg Val Ile Glu
            95                  100                 105

tgt gac gtg gtg aaa gac tac gcg ttt gtt cac atg gag aag gaa gca      508
Cys Asp Val Val Lys Asp Tyr Ala Phe Val His Met Glu Lys Glu Ala
        110                 115                 120

gat gcc aaa gcc gca atc gcg cag ctc aac ggc aaa gaa gtg aag ggc      556
Asp Ala Lys Ala Ala Ile Ala Gln Leu Asn Gly Lys Glu Val Lys Gly
    125                 130                 135

aag cgc atc aac gtg gaa ctc tcc acc aag ggt atg gtt ccg acc ggc      604
Lys Arg Ile Asn Val Glu Leu Ser Thr Lys Gly Met Val Pro Thr Gly
140                 145                 150                 155

gtt tag ccgagctctc tgattaccgc cgtttatcag agtcgcagct ttcgttccgc      660
Val

cgctcgccga caaagtcctc gctggattac cgtcgcctgc ccgatgccca ttccgattac 720
gcacgctatt cgggctccta taatgattac ctgcgggcgg ctcagatgca ctctggctac 780
cagcgccgca tgtagggcca tcctgggatg gggcaccaca gggagggagg gagaaaagag 840
gtgggtaggg ttacagatcc aggttataac tactctggcc catacctttc ctggttgtgg 900
tttttcatgc cctctaccat gtgggccttc cccaggagat gatcctgtta agtgttcggc 960
agtaacctac tttgttcctt cgcctcagca gcaaatcttg ctactggctc tagatctgcg 1020
gtttcccctc taccctgcct cccgtctccc cagaatggga atttctttta tgtttttatt 1080
tttttcctgg ctccctttta tttttgtgcg cgatatttaa ggtcgtctgg atggggaagc 1140
aacctgcagc tgaggtcgcc ggcgcctttt tctttttaga tgggaaggag gccaggaaag 1200
ggtcagctta accatttcct atgtgccaag ctgtgccagc agtccagggt accctgactg 1260
tccctctgta gactgttgag actgagttcc tgttgggaca gtcagttggt atgtatccaa 1320
gtccctgctg accactaatg ttctagctga tggtgagcgg cacagtccca cttccccatc 1380
tccccaagta ggtggtgtta gaaaacctta attttttttc ccttttgtat ggactacaaa 1440
taaaacttgg ggcaatttgc agtttggaaa cctggttgtc attgtcttga ttgcattcag 1500
tgtcaaagga gccaatttga catccaggaa gacatgatag ctaaagggaa ggcagtcaga 1560
gaacccagga ctggtgagga aaggggtttg aatgtcggaa ttagaaggat tgccctgtgg 1620
cccagaagga tttagctcat cgtcgtcctg ctagcctgcc atctttcaga ctttccaggg 1680
caggtggcct ggtcctcagc cctcaacttg agttagtcac accaaaatgc agtaaatgat 1740
gcccattttc ctctgcctgt gcttgaccat tttcactgac ttttctccag ttcaggaata 1800
acctgtgttc caatgggatg gttctggggt ttggcctctt aggaagtggg gaatgggctg 1860
ctgtggtatt tacaggctag tagatccttt aggcagtagg ctggaatgag tgctggggct 1920
gggacccaga aaggaaccag tagtggagtg ggggtaatgg cgttgcctga tactgcccta 1980
tggttggaat aaggaagggc agagaaaact tgaagcaggt atggggcatt ctggccatag 2040
agctcgtatt ttgcctgttg agctgtacca tggagcatcc tcctgtgtcc tgccaacccc 2100
tgccattata gtcaccggag actcctttac ctatccctat ctgttagggt tttcagatgt 2160
cctctcgctg ggtttatatt tgaaggtata gaatctggga aagggtgggg tgcaagctaa 2220
ccaaataggg atgctagggt tgggtgggga attgaggact cttctgtgct tttattgtag 2280
ggctggggat cgaggatctg ggcaaaaaat atgacatttc caaccaggga caaaatggag 2340
gcttgggctt agtgatgggt aaatggaaga ctggttgagt gggatgataa tgggagaact 2400
tactgatgct cttttgggaa aaggtgcttt aaagactcga tttgggagcc tactggggca 2460
aggcgactgg ggaggtgtac gtgttagtca agtggaggtc aggtgggtta tcttctgcct 2520
ccttcactgg cttcatgtga acttgcctgt gacagaatat cttgccctag atgtcctctt 2580

ccctcttgcc atcgtgcaaa agcattcgat cttatgaccg tgagttctta tttgtggatg 2640

ctaactgggg taatctcagg gagtactggg gccatggctc tttcccttgc ttctctttct 2700

gctcactgct ttgctttcag gtgcttagct gctccttttt atttctttgc aggactggga 2760

aagcatggga ggaggatttt gataagtttc ttgttctctg cgtggtgatg gggtggtctg 2820

agggccaagt taataagcct acctttggtt attaccctgg aaaattgagg gttgatggta 2880

gggttatgat tgtggcctgt gtatttgcaa tttttctttg tcagtgtggc tttctgtcat 2940

cttccttttt ctccagtgag ggttctgcca ctgtctcaat cccttggttc ctgcaaaaaa 3000

cagctaaatg tgctctcagg gccagtggtg atgttctgtg ttgcagcccc agctcttgaa 3060

agtgacctgg cccagatgag ctctgcctgc ttgtcatctt taggtcctgc tgcccctgct 3120

gcctcagccc tcaggctcct aactgctcaa gccagaagga gctacaaagc caaatcattt 3180

gtcatttttc tctggcagat tgttttaaaa tctccctccc ctcttttatt ttaattatgc 3240

cacctgtgtt aatcctatag cctgcttctt tgctttgtgg attactgatt tcaagttcta 3300

taacttgggg aaagttaggt tgagtgaatg ggaggacaga cactgaatgt gtgttttgga 3360

aaaatggact ctctatcttc agattattca gcttctccac ctatcacctc tacaacttgg 3420

ccttcattgg agaagtttta gttggcttat tcctggaaca agaccaccta gaggctttta 3480

gccttgtcag cctgccctct tctgaggttt ggaccttgtt ttaaattgtg tgcatggtgg 3540

gcttgagact ctcttgtaat ggaggtaatg ggactatcac tgaggggttc agcctatcca 3600

ggtaggctac cgttctagtc atacccatca cttaaattct gccacagtct gcacagacgg 3660

ttggtcctcc aggcatattg tcctattaag ccaaataggt ggacatcatg ggatggatga 3720

ggtcatcatg aaaacagatt tactggattt atggacctag tatatactat gtaacagatg 3780

gaaaactgaa ttttacaatc tataggtacc tttgaaaggt cataaagggt aaaagtctat 3840

agttttccag aaaataattc ttaaaactgg gttttatgtc tagatttaaa tcatgtatct 3900

gtctgtagtg cttcaaatgt ggccaggtgt ggtagctcac acctgtaatt cccagcgctt 3960

tgggaatttg aggcaagagg attgctagag gccaggattt tgagaccagc ttcggcaata 4020

cggtgagacc ttgtctctac caaaagaaaa gaaaaaaaaa a 4061

<210> 31
<211> 156
<212> PRT
<213> Homo sapiens

<400> 31

Met Lys Ile Phe Val Gly Asn Val Asp Gly Ala Asp Thr Thr Pro Glu
1               5                   10                  15

Glu Leu Ala Ala Leu Phe Ala Pro Tyr Gly Thr Val Met Ser Cys Ala
            20                  25                  30

Val Met Lys Gln Phe Ala Phe Val His Met Arg Glu Asn Ala Gly Ala
        35                  40                  45

Leu Arg Ala Ile Glu Ala Leu His Gly His Glu Leu Arg Pro Gly Arg
    50                  55                  60

Ala Leu Val Val Glu Met Ser Arg Pro Arg Pro Leu Asn Thr Trp Lys
65                  70                  75                  80

Ile Phe Val Gly Asn Val Ser Ala Ala Cys Thr Ser Gln Glu Leu Arg
                85                  90                  95

Ser Leu Phe Glu Arg Arg Gly Arg Val Ile Glu Cys Asp Val Val Lys
            100                 105                 110

Asp Tyr Ala Phe Val His Met Glu Lys Glu Ala Asp Ala Lys Ala Ala
        115                 120                 125

Ile Ala Gln Leu Asn Gly Lys Glu Val Lys Gly Lys Arg Ile Asn Val
    130                 135                 140

Glu Leu Ser Thr Lys Gly Met Val Pro Thr Gly Val
145                 150                 155

## Description

### Field of the invention

The present invention relates to agents that modulate CoAA expression and/or activity and the use thereof for the treatment of diseases. Said agents are preferably selected from the group consisting of CoAA or CoAM expression inhibitors, RBM14 splicing modulators, agents which interfere or promote class I HDAC recruitment by CoAA and their combinations. Said agents are mainly intended for the treatment of viral diseases and/or cancer

### Background

Acquired immune deficiency syndrome (AIDS) can be caused by two human immunodeficiency viruses, type 1 (HIV-1) and type 2 (HIV-2). HIV-1 (group M) originated from simian immunodeficiency virus chimpanzee (SIVcpz) and is responsible for the AIDS pandemic. HIV-2 arose from SIVsm (sooty mangabey) and is mainly found in West Africa. In addition to differences in geographic distribution, dramatic differences exist in genomic organization, transmission and progression to AIDS. The proviral loads found in peripheral blood mononuclear cells (PBMCs) are usually similar in people infected with HIV-1 and people infected with HIV-2. In contrast, the viral loads observed for HIV-2 are invariably lower than those observed with HIV-1 infections. Moreover, while most HIV-1 cohort studies depict 5-15% of their subjects to fit a definition of long-term non-progression, 86-95% of HIV-2 infected individuals are similarly classified. Progression is also much slower in HIV-2 diseased individuals compared to HIV-1-infected patients, as evidenced by slower rates of CD4+ T-cell depletion and progression to AIDS. The molecular bases for these differences remain largely unknown and comparative studies are clearly required to better understand them.

During HIV-1 infection, a pool of resting CD4+ T cells remains infected by the virus in a latent form. However, latent virus can not be targeted by antiretroviral therapies. New strategies under investigation focus on re-inducing the expression of latent HIV-1. For example, Archin et al. (Aids Research and Human Retroviruses, 2009, 25:207-212) use SAHA (*SuberoylAnilide Hydroxamic Acid*), a potent inhibitor of class I HDACs, to induce latent HIV. HDACs activity is indeed involved in the proviral quiescence in resting CD4+ cells.

New drugs are still needed in the treatment of viral diseases in latent form, particularly HIV-1 disease, said drugs being able to re-induce the virus so that infected cells become sensible to classical therapies.

CoAA (*CoActivator Activator*) is a heterogeneous nuclear ribonucleoprotein (hnRNP)-like protein that mediates both transcription and splicing in a promoter-preferential manner (Auboeuf et al., Molecular and Cellular Biology, 2004, 24: 442-453).

Kang and al. (Cancer Research, 2008, 68:7887-7896) disclose that CoAA inhibits transcription of the c-Myc gene by recruiting HDAC3 and that said CoAA could act as a tumor suppressor in renal carcinoma. This article discloses the only known example of HDAC recruitment by CoAA, whereas HDACs regulate many genes according to different mechanisms.

US2008/241140 discloses that amplifications in the CoAA gene block stem cell differentiation and induce cancer stem cells. US2008/241140 thus provides compositions that antagonize or interfere with the biological activity of CoAA, for treating cancer. Said compositions preferably comprise inhibitory nucleic acids that block expression of CoAA amplicon.

Controversial results are thus disclosed in the state of the art relating to the role of CoAA in cancer.

### Description of the invention

The inventors designed a framework based on the computational reconstruction of meta-regulation networks able to infer novel knowledge in HIV biology from large sets of expression data. This strategy used miRNA target predictions as "computational filters" and helped narrow down transcriptomics data to few interesting candidates: MDK, RBM14 and CNNM4). Interestingly, none of these genes was retrieved by other large-scale approaches such as RNAi screens.

RBM14 gene, also known as PSP2, encodes a transcriptional and a RNA splicing modulator. Alternatively spliced transcript variants encoding different isoforms are described for RBM14 gene, in particular CoAA and CoAM, which harbor opposing effects on transcription (Iwasaki et al., J Biol Chem., 2001, 276:33375-33383).

Surprisingly, the Inventors have shown that CoAA inhibits HIV transcription by the recruitment of HDACs on HIV-1-LTR and that HDACs are required for CoAA-mediated inhibition of HIV transcription. As a matter of fact, CoAA binds HIV LTR and is therefore present at the HIV-1 transcription site where HDACs are then recruited.

HDACs (*Histone deacetylases*) are a class of enzymes that allow histones to wrap the DNA more tightly by selectively deacetylating the ε-amino groups of lysine located near the amino termini of core histone proteins. Chromatin deacetylation correlates with gene silencing.

Interestingly, the Inventors have shown that CoAA does not require the viral transactivator Tat to inhibit HIV transcription.

Besides, new compounds able to modulate CoAA activity were found. For example, in the presence of L-glutamine, CoAA promotes transcription, whereas in the presence of L-alanyl-L-glutamine, CoAA represses transcription.

The present invention is mainly based on the modulation of CoAA expression and/or activity to modulate viral transcription.

For that purpose, the inventors also disclose new agents able to modulate CoAA expression and/or activity.

Thanks to their specific mode of action, these new agents have fewer side effects.

In a first aspect, the present invention is intended for the treatment of latent viral diseases, wherein there is a need to induce the virus, in order to be able to eradicate the virus by classical antiviral therapies.

In another aspect, the present invention may also be used for the treatment of any disease that needs a repression of transcription, such as viral diseases in proliferative form and cancer.

The present invention may thus be used for the treatment of any disease that needs a modulation of transcription in a target cell, said modulation being either a repression or an activation of the transcription.

The target cell is a cell of a subject to be treated.

The cell of a subject to be treated may be a healthy cell, but is preferably an infected cell or an abnormal cell, such as a cancer cell.

The subject to be treated is an animal or a human, preferably a human.

When the subject to be treated is an animal, it is preferably a non-human mammal, for example a mouse, rat, cat, dog, rabbit or primate.

The infected cell is preferably a cell infected by a virus.

The present invention particularly relates to an agent which modulates CoAA expression and/or activity for use in the treatment of a disease selected from the group consisting of viral diseases and cancer.

In the context of the invention, the term "treatment" is used herein to characterize a therapeutic method or process that is aimed at (1) slowing down or stopping the progression, aggravation, or deterioration of the symptoms of the disease state or condition to which such term applies; (2) alleviating or bringing about ameliorations of the symptoms of the disease state or condition to which such term applies; and/or (3) reversing or curing the disease state or condition to which such term applies.

The treatment of a viral disease in latent form according to the present invention comprises a step of re-activation of the virus.

The terms "virus activation", "virus re-activation" and "virus induction" are herein synonymous.

The viral diseases that may be treated according to the invention may be caused be a virus from a family selected from the group consisting of the *Retroviridae* family, the *Herpesviridae* family, the *Togaviridae* family, the *Hepadnaviridae* family or the *Adenoviridae* family.

A virus of the *Retroviridae* family is for example HIV (*Human Immunodeficiency Virus*), preferably HIV-1 or HIV-2, or HTLV (*Human T-Lymphotropic Virus*), preferably HTLV-1, HTLB-2, HTLV-3 or HTLV-4.

A virus of the *Herpesviridae* family is for example chosen among HSV, EBV, VZV, CMV, HHV-6, HHV-7 or KSHV.

HSV (also called *Herpes Simplex Virus*) is preferably chosen among HSV-1 (also called HHV-1) or HSV-2 (also called HHV-2).

EBV means Epstein-Barr Virus, also called HHV-4.

VZV means Varicella Zoster Virus.

CMV means Cytomegalovirus, also called HHV-5.

KSHV means Kaposi's Sarcoma-associated Herpes Virus.

A virus of the *Togaviridae* family is for example Rubella.

A virus of the *Hepadnaviridae* family is for example HBV (also called *Hepatitis B Virus*)*.*

A virus of the *Adenoviridae* family is for example AdV 40 (also called Adenovirus type 40) or AdV 41 (also called Adenovirus type 41).

As used herein, RBM14 denotes any mammalian form of RBM14, in particular a human, mouse, rat, cat, dog, rabbit or primate form of RBM14.

In human, the RBM14 gene is located on chromosome 11q13.2. A reference sequence for human RBM14 gene is for example the sequence of 20353 nucleotides shown in sequence SEQ ID NO: 27 (database access number NCBI: NG_030394.1, as available on 08/07/2013).

Any naturally occurring variant thereof, in particular polymorphic variant, is encompassed within the definition of human RBM14 gene.

The sequence of the RBM14 gene according to the invention may be the sequence SEQ ID NO: 27 or a sequence at least 80% identical to sequence SEQ ID NO: 27, preferably at least 85% identical to sequence SEQ ID NO: 27, more preferably at least 90% identical to sequence SEQ ID NO: 27, still more preferably at least 95% identical to sequence SEQ ID NO: 27 or at least 99% identical to sequence SEQ ID NO: 27.

The CoAA protein, also called CoActivator Activator, is one isoform product of the RBM14 gene.

As used herein, CoAA denotes any mammalian form of CoAA, in particular a human, mouse, rat, cat, dog, rabbit or primate form of CoAA.

A reference sequence for human CoAA protein is for example the protein of 669 amino acids shown in sequence SEQ ID: 29 (database access number NCBI: NM_006328.3, as available on 06/05/2013).

A reference sequence for a human CoAA cDNA is for example shown in sequence SEQ ID: 28 (database access number NCBI: NM_006328.3, as available on 06/05/2013).

The CoAA mRNA sequence corresponds to the CoAA cDNA sequence wherein thymine bases are replaced by uracil bases.

The sequence of the CoAA mRNA according to the invention may be the sequence SEQ ID NO: 28 wherein thymine bases are replaced by uracil bases, or a sequence at least 80% identical, preferably at least 85% identical, more preferably at least 90% identical, still more preferably at least 95% identical or at least 99% identical to sequence SEQ ID NO: 28 wherein thymine bases are replaced by uracil bases.

The CoAM protein, also called CoActivator Modulator, is another isoform product of the RBM14 gene.

As used herein, CoAM denotes any mammalian form of CoAM, in particular a human, mouse, rat, cat, dog, rabbit or primate form of CoAM.

A reference sequence for human CoAM protein is for example the protein of 156 amino acids shown in sequence SEQ ID: 31 (database access number NCBI: NM_001198836.1, as available on 12/05/13).

A reference sequence for a human CoAM cDNA is for example shown in sequence SEQ ID: 30 (database access number NCBI: NM_001198836.1, as available on 12/05/13).

The CoAM mRNA sequence corresponds to the CoAM cDNA sequence wherein thymine bases are replaced by uracil bases.

The sequence of the CoAM mRNA according to the invention may be the sequence SEQ ID NO: 30 wherein thymine bases are replaced by uracil bases, or a sequence at least 80% identical, preferably at least 85% identical, more preferably at least 90% identical, still more preferably at least 95% identical or at least 99% identical to sequence SEQ ID NO: 30 wherein thymine bases are replaced by uracil bases.

As used herein, "a first amino acid / nucleotide sequence is at least x% identical to a second amino acid / nucleotide sequence" means that x% represents the number of amino acids / nucleotides in the first sequence which are identical to their matched amino acids / nucleotides of the second sequence when both sequences are optimally aligned, relative to the total length of the second amino acid / nucleotide sequence. Both sequences are optimally aligned when x is maximum. The alignment and the determination of the percentage of identity may be carried out manually or automatically using for instance the Needle program which is based on the Needleman and Wunsch algorithm, described in Needleman and Wunsch (1970) J. Mol Biol. 48:443-453, with for example the following parameters for polypeptide sequence comparison: comparison matrix: BLOSUM62, gap open penalty: 10 and gap extend penalty: 0.5, end gap penalty: false, end gap open penalty = 10, end gap extend penalty = 0.5; and the following parameters for polynucleotide sequence comparison: comparison matrix: DNAFULL; gap open penalty = 10, gap extend penalty = 0.5, end gap penalty: false, end gap open penalty = 10, end gap extend penalty = 0.5.

The present invention more particularly relates to an agent which modulates CoAA expression and/or activity for the use in the treatment of a disease selected from the group consisting of viral diseases and cancer, wherein said agent modulates class I HDAC recruitment by CoAA.

Said agent modulates the recruitment by CoAA of at least one HDAC of class I selected from the group consisting of HDAC1, HDAC2, HDAC3 and HDAC8.

Said agent which modulates CoAA expression and/or activity may modulate the recruitment by CoAA of at least two, more preferably at least 3 HDACs of class I selected from the group consisting of HDAC1, HDAC2, HDAC3 and HDAC8.

In one embodiment, the agent of the invention which modulates CoAA expression and/or activity modulates the recruitment of HDAC1, HDAC2, HDAC3 and HDAC8.

In one preferred embodiment, the present invention relates to an agent which modulates CoAA expression and/or activity for the use as defined above in the treatment of a viral disease in latent form.

A viral disease in latent form is a viral disease wherein the virus is latent within the infected cells.

By "latent virus" or "non active virus" or "dormant virus", one means a virus whose genome is still present in the infected cells, without producing any viral particles.

The agent which modulates CoAA expression and/or activity for use in the treatment of a viral disease in latent form has at least one of the following properties:
- inhibiting CoAA expression, particularly inhibiting the expression of CoAA protein or the translation of CoAA mRNA,
- increasing CoAM expression, particularly increasing the splicing towards CoAM isoform, and/or
- modulating CoAA activity, resulting in interfering with class I HDAC recruitment by CoAA.

The present invention thus relates to an agent for the use as define above, wherein said disease is a viral disease in latent form and wherein said agent is selected from the group consisting of a CoAA expression inhibitor, a RBM14 splicing modulator that increases splicing towards CoAM protein, an agent which interferes with class I HDAC recruitment by CoAA and their combinations.

A "CoAA expression inhibitor" according to the invention inhibits expression of CoAA protein, in particular by inhibiting the translation of the CoAA mRNA.

By "CoAA mRNA", one means the mature mRNA encoding CoAA protein obtained after splicing of RBM14 pre-mRNA. RBM14 pre-mRNA is itself obtained by transcription of the RBM14 gene.

As used herein, an "agent which interferes with class I HDAC recruitment by CoAA" modulates CoAA protein activity in a target cell, resulting in interfering with class I HDAC recruitment by CoAA.

The present invention particularly relates to an agent for the use as defined above, wherein said CoAA expression inhibitor is selected from the group consisting of siRNA, shRNA, miRNA, dsRNA, aptamer, antisense nucleic acid, peptide nucleic acid, antibody and their combinations.

In one embodiment, a siRNA, shRNA, miRNA, dsRNA, aptamer, antisense nucleic acid, peptide nucleic acid or antibody that is a CoAA expression inhibitor specifically binds to a target sequence within the CoAA mRNA, thus preventing the expression of CoAA protein.

The CoAA expression inhibitor specifically binds to a target sequence within the CoAA mRNA, but does not bind to the mRNA of the other isoforms of the RBM14 gene, such as CoAM mRNA.

The target sequence of CoAA mRNA is for example a region of the CoAA mRNA of sequence SEQ ID NO: 28 comprising or consisting of nucleotides 1140 to 1175, preferably nucleotides 1147 to 1169, more preferably nucleotides 1149 to 1167.

In one preferred embodiment, a CoAA expression inhibitor according to the invention specifically hybridizes to one of the following sequences: CCCUUGCCUCCUAUGGUAA (SEQ ID NO: 1) or CGCUAUUCGGGCUCCUAUA (SEQ ID NO: 2).

Sequence SEQ ID NO: 1 corresponds to nucleotides 1149 to 1167 of the sequence SEQ ID NO: 28, wherein thymine bases are replaced by uracil bases.

Sequence SEQ ID NO: 2 corresponds to nucleotides 2078 to 2096 of the sequence SEQ ID NO: 28, wherein thymine bases are replaced by uracil bases.

A siRNA, shRNA, miRNA, dsRNA, aptamer, antisense nucleic acid, peptide nucleic acid or antibody that is a CoAA expression inhibitor is able to decrease the expression of the CoAA protein by at least 10%, at least 20%, at least 30%, at least 40 %, more preferably by at least 50%, at least 60%, at least 70 %, and most preferably by at least 75%, at least 80%, at least 85%, at least 90%, at least 95%, at least 96%, at least 97%, at least 98%, at least 99%, or 100%.

The expression of the CoAA protein may be measured by any suitable method well known by the skilled person, such as Western blot, ELISA or indirect methods, for example RT-qPCR (*Reverse-Transcription - quantitative PCR*).

The percentage of CoAA protein expression decrease is obtained by measuring CoAA protein expression in a sample treated with a CoAA expression inhibitor and CoAA protein expression in a control sample placed in the same conditions as the treated sample, except the presence of said CoAA expression inhibitor.

The sample may be a cell, preferably a human cell or an animal cell, or a tissue.

The sample may be a healthy cell, an infected cell, an abnormal cell, such as a cancer cell, an immortalized cell, or a cell from a cellular line.

A "siRNA", also called "small interfering RNA" or "short interfering RNA" is a short-length double strand RNA.

A siRNA according to the invention consists for example in 15 to 50 base pairs, more preferably 18 to 30 base pairs.

In some embodiments, the siRNA can be of 15, 16, 17, 18, 19, 20, 21, 22, 23, 24, 25, 26, 27, 28, 29, 30 base pairs in length.

A preferred siRNA according to the invention consists in 19 base pairs complementary to the target sequence and a 3' overhang of two nucleotides complementary to the target sequence, said 3' overhang being present on each strand of the siRNA.

A preferred CoAA siRNA according to the invention comprises or consists of a sequence that hybridizes to the following sequence CCCUUGCCUCCUAUGGUAA (SEQ ID NO: 1).

For example, a preferred CoAA siRNA according to the invention has one strand comprising or consisting of sequence CCCUUGCCUCCUAUGGUAA (SEQ ID NO: 1) and preferably a 3' overhang of two nucleotides, as defined above.

Another preferred CoAA siRNA according to the invention comprises or consists of a sequence that hybridizes to the following sequence: CGCUAUUCGGGCUCCUAUA (SEQ ID NO: 2).

For example, another preferred CoAA siRNA according to the invention has one strand comprising or consisting of sequence CGCUAUUCGGGCUCCUAUA (SEQ ID NO: 2) and preferably a 3' overhang of two nucleotides, as defined above.

The present invention particularly relates to a CoAA expression inhibitor selected from the group consisting of a siRNA comprising or consisting of a sequence hybridizing to sequence SEQ ID NO :1 or SEQ ID NO : 2.

As used herein, "a sequence that hybridizes to a given sequence" means a sequence that hybridizes to said given sequence under moderate or high stringency conditions.

Stringent conditions or high stringency conditions may be identified by those that: (1) employ low ionic strength and high temperature for washing, for example 0,015 M sodium chloride/0,0015 M sodium citrate/0,1% sodium dodecyl sulfate at 50°C; (2) employ during hybridization a denaturing agent, such as formamide, for example, 50% (v/v) formamide with 0,1% bovine serum albumin/0,1% Ficoll/0,1% polyvinylpyrrolidone/50 mM sodium phosphate buffer at pH 6,5 with 750 mM sodium chloride, 75 mM sodium citrate at 42°C; or (3) employ 50% formamide, 5 x SSC (0,75 M NaCl, 0,075 M sodium citrate), 50 mM sodium phosphate (pH 6,8), 0,1% sodium pyrophosphate, 5 x Denhardt's solution, sonicated salmon sperm DNA (50 µg/ml), 0,1% SDS, and 10% dextran sulfate at 42°C, with washes at 42°C in 0,2 x SSC (sodium chloride/sodium citrate) and 50% formamide at 55°C, followed by a high-stringency wash consisting of 0,1 x SSC containing EDTA at 55°C.

Moderate stringency conditions may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent than those described above.

An example of moderate stringency conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C.

The present invention more particularly relates to a CoAA expression inhibitor selected from the group consisting of a siRNA having one strand comprising or consisting of sequence SEQ ID NO :1, SEQ ID NO : 2, a variant of sequence SEQ ID NO: 1, a variant of sequence SEQ ID NO: 2 and their combinations.

A variant of sequence SEQ ID NO: 1 is a sequence at least 80% identical to sequence SEQ ID NO: 1 and which is able to hybridize CoAA mRNA sequence. Preferably, a variant of sequence SEQ ID NO: 1 is a sequence at least 80%, 81%, 82%, 83%, 84%, 85%, 86%, 87%, 88%, 89%, 90%, 91%, 92%, 93%, 94%, 95%, 96%, 97%, 98%, 99% identical to sequence SEQ ID NO: 1 and which is able to hybridize CoAA mRNA sequence.

A variant of sequence SEQ ID NO: 2 is a sequence at least 80% identical to sequence SEQ ID NO: 2 and which is able to hybridize CoAA mRNA sequence. Preferably, a variant of sequence SEQ ID NO: 2 is a sequence at least 80%, 81%, 82%, 83%, 84%, 85%, 86%, 87%, 88%, 89%, 90%, 91%, 92%, 93%, 94%, 95%, 96%, 97%, 98%, 99% identical to sequence SEQ ID NO: 2 and which is able to hybridize CoAA mRNA sequence.

The present invention also relates to an agent for the use as defined above, wherein said agent is a CoAA siRNA comprising or consisting of a sequence hybridizing to sequence SEQ ID NO :1 or SEQ ID NO : 2

The present invention also relates to an agent for the use as defined above, wherein said agent is a CoAA siRNA having on strand comprising or consisting of sequence SEQ ID NO: 1, sequence SEQ ID NO: 2, a variant of sequence SEQ ID NO: 1, a variant of sequence SEQ ID NO: 2 and their combinations.

As used herein, "shRNA", also called "small hairpin RNA" or "short hairpin RNA", refers to a RNA sequence that makes a tight hairpin turn.

The loop of the shRNA can vary in length.

In some embodiments the loop is 5, 6, 7, 8, 9, 10, 11, 12 or 13 nucleotides in length.

The hairpin structure can also contain 3 or 5 overhang portions.

In some embodiments, the overhang is a 3' or a 5' overhang of 1, 2, 3, 4, or 5 nucleotides in length.

In some embodiments, there is no overhang.

As used herein, "miRNA", also called "microRNA", is a small non-coding RNA molecule found in plants and animals.

As used herein, "dsRNA" or "Long double-stranded RNA" refers to an oligoribonucleotide or polyribonucleotide, modified or unmodified, and fragments or portions thereof, of genomic or synthetic origin or derived from the expression of a vector, which may be partly or fully double stranded and which may be blunt ended or contain a 5' and or 3' overhang, and also may be of a hairpin form comprising a single oligoribonucleotide which folds back upon itself to give a double stranded region.

In some embodiments, the dsRNA has a size ranging from 150 bp to 3000 bp, preferably ranging from 250 bp to 2000 bp, still more preferably ranging from 300 bp to 1000 bp.

As used herein, "aptamer" refers to a small single-stranded nucleic acid that folds into a well-defined three-dimensional structure.

As used herein, "antisense nucleic acid" refers to a RNA or DNA molecule that is complementary to the target sequence.

As used herein, "peptide nucleic acid", also called "PNA" refers to a synthetic analog of DNA or RNA whose backbone is composed of repeating N-(2-aminoethyl)-glycine units linked by peptide bonds.

As used herein, "antibody" refers to immunoglobulin molecules and immunologically active portions of immunoglobulin molecules, i.e. molecules that contain an antigen binding site which immunospecifically binds an antigen, the antigen being the target sequence according to the invention. As such, the term antibody encompasses not only whole antibody molecules, but also antibody fragments as well as variants of antibodies, including derivatives such as humanized antibodies.

For example, an antibody according to the invention includes Fab (*Fragment antigen binding*), Fv, scFv (Single Chain Fv), Fc (*Fragment crystallizable*), F(ab')2, and any peptide comprising one or several CDRs (*Complementarity Determining Region*).

The CoAA inhibitor may be synthesized chemically or expressed via the use of expression vector or equivalent thereof using protocols known in the art.

The RBM14 splicing modulator may be selected from the group consisting of spliceostatin A, borrelidin, amilorid, a steroid hormone, for example progesterone, and their combinations.

For the first time, the inventors have shown that CoAA protein activity can be modulated in the presence of particular compounds.

For example, in the presence of L-glutamine, CoAA protein promotes transcription.

The present invention thus also relates to an agent for the use as defined above, wherein said agent which interferes with class I HDAC recruitment by CoAA is L-glutamine.

The viral disease may be caused by any virus that can be in a latent form.

Said virus in latent form may be a virus of a family as defined above, particularly selected from the group consisting of the *Retroviridae* family, the *Herpesviridae* family, the *Togaviridae* family, the *Hepadnaviridae* family or the *Adenoviridae* family.

The present invention particularly relates to an agent for the use as defined above, wherein said viral disease in latent form is caused by a virus selected from the group consisting of HIV-1, HTLV-1, Rubella, HBV, a virus of the *Herpesviridae* family, AdV 40 and AdV 41.

In another embodiment, the present invention relates to an agent which modulates CoAA expression and/or activity for the use as defined above in the treatment of a viral disease in proliferative form and/or of cancer.

A viral disease in proliferative form is a viral disease wherein the virus replicates and viral RNA and proteins are expressed. New viral particles may also be produced, said new viral particles thus infecting new cells.

The agent which modulates CoAA expression and/or activity for the use in the treatment of a viral disease in proliferative form and/or of cancer has at least one of the following properties:
- inhibiting CoAM expression, particularly inhibiting CoAM expression or translation of CoAM mRNA,
- increasing CoAA expression, particularly increasing the splicing towards CoAA isoform, and/or
- modulating CoAA activity, resulting in promoting class I HDAC recruitment by CoAA.

The present invention particularly relates to an agent for the use as defined above, wherein said disease is a viral disease in proliferative form and/or cancer and wherein said agent is selected from the group consisting of a CoAM expression inhibitor, a RBM14 splicing modulator that increases splicing towards CoAA protein, an agent which promotes class I HDAC recruitment by CoAA and their combinations.

A "CoAM expression inhibitor" according to the invention inhibits the expression of CoAM protein, in particular by inhibiting the translation of the CoAM mRNA.

By "CoAM mRNA", one means the mature mRNA encoding CoAA protein obtained after splicing of RBM14 pre-mRNA.

As used herein, an "agent which promotes class I HDAC recruitment by CoAA" modulates CoAA protein activity in a target cell, resulting in promoting class I HDAC recruitment by CoAA.

The present invention particularly relates to an agent for the use as defined above, wherein said CoAM expression inhibitor is selected from the group consisting of siRNA, shRNA, miRNA, dsRNA, aptamer, antisense nucleic acid, peptide nucleic acid, antibody and their combinations.

In one embodiment, a siRNA, shRNA, miRNA, dsRNA, aptamer, antisense nucleic acid, peptide nucleic acid or antibody that is a CoAM expression inhibitor specifically binds to a target sequence within the CoAM mRNA, thus preventing the expression of CoAM protein.

The CoAM expression inhibitor specifically binds to a target sequence within the CoAM mRNA, but does not bind to the mRNA of the other isoforms of the RBM14 gene, such as CoAA mRNA.

A siRNA, shRNA, miRNA, dsRNA, aptamer, antisense nucleic acid, peptide nucleic acid or antibody that is a CoAM expression inhibitor is able to decrease the expression of the CoAM protein by at least 10%, at least 20%, at least 30%, at least 40 %, more preferably by at least 50%, at least 60%, at least 70 %, and most preferably by at least 75%, at least 80%, at least 85%, at least 90%, at least 95%, at least 96%, at least 97%, at least 98%, at least 99%, or 100%.

The expression of the CoAM protein may be measured by any suitable method well known by the skilled person, such as Western blot, ELISA or indirect methods, for example RT-qPCR (*Reverse-Transcription - quantitative PCR*).

The percentage of CoAM protein expression decrease is obtained by measuring CoAM protein expression in a sample treated with a CoAM expression inhibitor and CoAM protein expression in a control sample placed in the same conditions as the treated sample, except the presence of said CoAM expression inhibitor.

The sample may be a cell, preferably a human cell or an animal cell, or a tissue.

The sample may be a healthy cell, an infected cell, an abnormal cell, such as a cancer cell, an immortalized cell, or a cell from a cellular line.

The RBM14 splicing modulator may be selected from the group consisting of spliceostatin A, borrelidin, amilorid, a steroid hormone, for example progesterone, and their combinations.

As explained above, the inventors have shown that CoAA protein activity can be modulated in the presence of particular compounds.

For example, in the presence of L-Alanyl-L-Glutamine, CoAA protein represses transcription, particularly by promoting class I HDAC recruitment.

The present invention thus relates to an agent for the use as defined above, wherein said agent which promotes class I HDAC recruitment by CoAA is L-alanyl-L-glutamine.

Said virus in proliferative form may be a virus of a family as defined above, particularly selected from the group consisting of the *Retroviridae* family, the *Herpesviridae* family, the *Togaviridae* family, the *Hepadnaviridae* family and the *Adenoviridae* family.

The present invention particularly relates to an agent for the use as defined above, wherein said viral disease in proliferative form is caused by a virus selected from the group consisting of HIV-2, HIV-1, HTLV-1, Rubella, HBV, a virus of the *Herpesviridae* family, AdV 40 and AdV 41, and/or in that said cancer is selected from the group consisting of lung cancer, pancreas cancer, melanoma, esophageal cancer, lymphoma, squamous skin cancer, stomach cancer and ovary cancer.

The agent which modulates CoAA expression and/or activity according to the invention may be formulated in a pharmaceutical composition.

A pharmaceutical composition according to the invention comprises at least one agent which modulates CoAA expression and/or activity and at least one pharmaceutically acceptable vehicle which is not prejudicial to the subject to be treated.

Pharmaceutically acceptable vehicles that may be used in the compositions of this invention include, but are not limited to, ion exchangers, alumina, aluminium stearate, lecithin, self-emulsifying drug delivery systems (SEDDS) such as d-a-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

As appreciated by skilled artisans, pharmaceutical compositions are suitably formulated to be compatible with the intended route of administration. Examples of suitable routes of administration include parenteral route, including for instance intramuscular, subcutaneous, intravenous, intraperitoneal or local intratumoral injections.

The oral route can also be used, provided that the composition is in a form suitable for oral administration, able to protect the active principle from the gastric and intestinal enzymes.

For siRNA, shRNA, miRNA, dsRNA, aptamer, antisense nucleic acid and peptide nucleic acid administration, the pharmaceutical compositions of the invention may be prepared in forms that include encapsulation in liposomes, microparticles, microcapsules, nanocapsules and lipid-based carrier systems.

The siRNA, shRNA, miRNA, dsRNA, aptamer, antisense nucleic acid and peptide nucleic acid of the present invention can also be delivered as a naked molecule.

Expression vectors may also be used to continually express a siRNA, shRNA, miRNA, dsRNA, aptamer, antisense nucleic acid or antibody in transiently and stably transfected mammalian cells (see for example, e.g., Brummelkamp et al., 2002, Science, 296:550-553; Paddison et al., 2002, Genes & Dev, 16:948-958).

The present invention also relates to a pharmaceutical composition comprising an agent which modulates CoAA activity and/or expression according to the invention, for use as defined above in the treatment of a viral disease in latent form, or a viral disease in proliferative form and/or cancer.

The pharmaceutical composition according to the invention comprises a therapeutically effective amount of the agent which modulates CoAA activity and/or transcription.

A therapeutically effective amount is an amount sufficient to achieve modulation of CoAA activity and/or expression or to treat the desired disease without causing overly negative effects in the subject to which the composition is administered.

The exact amount of the agent to be used will vary according to the age and the weight of the subject to be treated, the type of disease, the mode of administration, the frequency of administration as well as the other ingredients in the composition which comprises the agent.

Effective doses will also vary depending on route of administration, as well as the possibility of co-usage with other agents.

The present invention also relates to an agent for the use as defined above in combination with at least one antiviral drug and/or at least one anti-cancer drug.

Said antiviral drug and/or anti-cancer drug to be used in combination with the agent of the invention is intended to be administered simultaneously or sequentially.

The present invention particularly relates to an agent which modulates CoAA expression and/or activity as defined above for use in the treatment of HIV, in combination with an antiretroviral therapy.

An antiretroviral therapy may for example comprise zidovudine, lamivudine, emtricitabine, didanosine, stavudine, abacavir, zalcitabine, tenofovir, racivir, amdoxovir, apricitabine, elvucitabine, efavirenz, nevirapine, etravirine, delavirdine, rilpivirine, fosalvudine, tipranavir, indinavir, saquinavir, fosamprenavir, ritonavir, darunavir, atazanavir, nelfinavir, kaletra, lopinavir, elvitegravir, dolutegravir, vicriviroc, cobicistat, TNX-355 or their combinations.

Another object of the invention is a composition comprising at least one agent which modulates CoAA expression and/or activity and at least one drug, wherein said drug is an antiviral drug and/or an anti-cancer drug.

In one embodiment, the composition comprises at least one agent which modulates CoAA expression and/or activity and at least one drug, wherein said agent is selected from the group consisting of a CoAA expression inhibitor, a RBM14 splicing modulator that increases splicing towards CoAM protein, an agent which interferes with class I HDAC recruitment by CoAA and their combinations, and wherein said drug is an antiviral drug.

In another embodiment, the composition comprises at least one agent which modulates CoAA expression and/or activity and at least one drug, wherein said agent is selected from the group consisting of a CoAM expression inhibitor, a RBM14 splicing modulator that increases splicing towards CoAA protein, an agent which promotes class I HDAC recruitment by CoAA and their combinations, and wherein said drug is an antiviral drug and/or an anti-cancer drug.

The present invention also relates to a kit comprising:
- at least one agent which modulates CoAA expression and/or activity selected from the group consisting of a CoAA expression inhibitor, a RBM14 splicing modulator that increases splicing towards CoAM protein, an agent which interferes with class I HDAC recruitment by CoAA and their combinations, and
- at least one antiviral drug,
for simultaneous, separate or sequential use in the treatment of a viral disease in latent form.

The present invention also relates to a kit comprising:
- at least one agent which modulates CoAA expression and/or activity selected from the group consisting of a CoAM expression inhibitor, a RBM14 splicing modulator that increases splicing towards CoAA protein, an agent which promotes class I HDAC recruitment by CoAA and their combinations, and
- at least one drug selected among an antiviral drug and/or an anti-cancer drug, for simultaneous, separate or sequential use in the treatment of a viral disease in proliferative form and/or of cancer.

According to another embodiment, the present invention relates to a method for the treatment of a viral disease in latent form, or a viral disease in proliferative form and/or of cancer, comprising the administration to a patient in need thereof of a therapeutically effective amount of an agent which modulates CoAA expression and/or activity as defined above or of a pharmaceutical composition as defined above.

The present invention will be further illustrated in view of the following figures and examples.

### Brief description of the figures

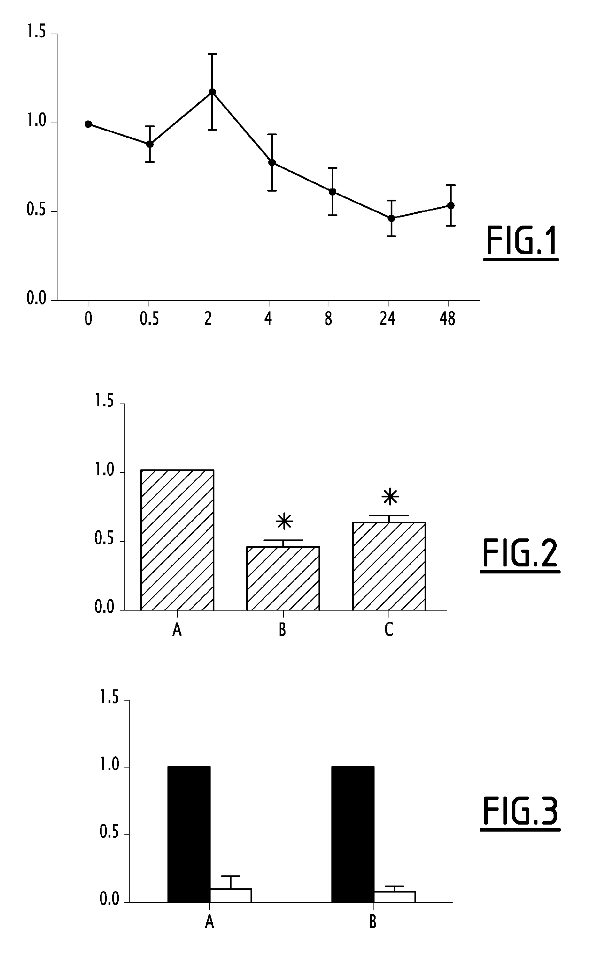

Figure 1: RT-qPCR analyses of CoAA mRNA performed in Jurkat T-cells infected with HIV-1 NL4.3 for 48 hours. Results were normalized with GAPDH mRNA and to the results obtained at day 0 (fold change=1). Ordinate: fold change. Abscissa: hours post-infection.
Figure 2: ELISA directed against the HIV-1 and HIV-2 core protein performed in the supernatant of Jurkat T-cells transfected with a CoAA-GFP-expressing vector and infected with HIV-1NL4.3 (B) or HIV-2ROD (C). Results (relative p24 dosage) were normalized to that obtained with the control GFP-expressing vector (A). *, pval < 0.01 (t-test comparing core protein dosage in the absence and presence of CoAA).
Figure 3: RT-qPCR analyses of spliced (A - left) and unspliced (B - right) HIV-1 mRNAs performed in Jurkat T-cells transfected with a CoAA-GFP-expressing vector (white bars) and infected with HIV-1 NL4.3. Results were normalized to that obtained with the control GFP-expressing vector (black bars). Ordinate: fold change.

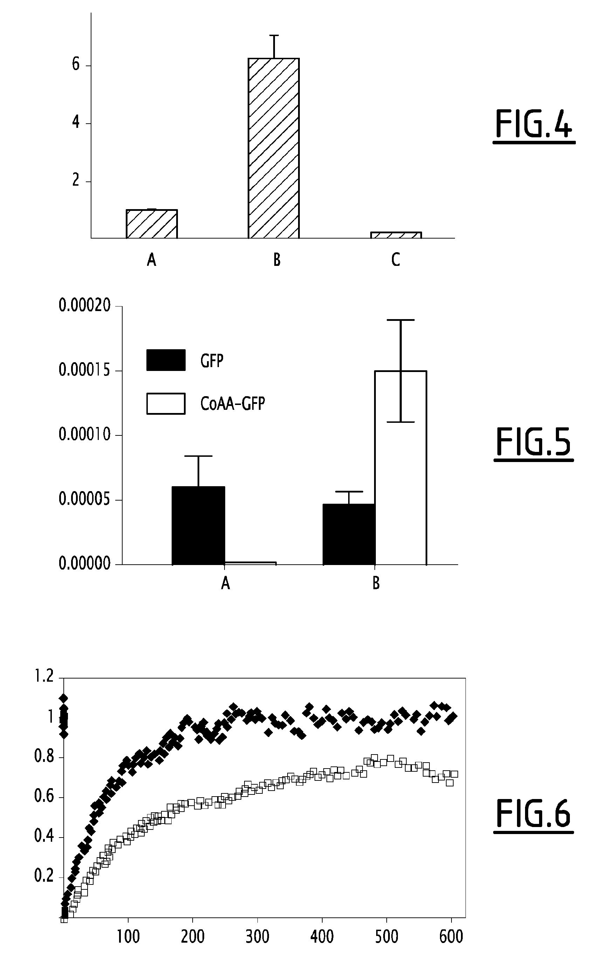

Figure 4: RT-qPCR analyses of HIV-1 reporter RNA performed in the 2E11 HIV-1 reporter cells and transfected with a CoAA-GFP-expressing vector (forced expression - C) or siRNAs directed against CoAA (B). Results were normalized with GAPDH mRNA and to the results obtained with a control GFP-expressing vector (for forced expression) and control siRNAs (for RNAi) (C). Ordinate: fold change.
Figure 5: ChIP experiments performed in Jurkat T-cells transfected with a CoAA-GFP-expressing vector (white bars) and infected with HIV-1NL4.3 (B). Results are indicated as percentage of input and were normalized to that obtained with the control GFP-expressing vector (black bars). An irrelevant promoter sequence was used as a negative control (A). Ordinate: % input.
Figure 6. FRAP experiments performed in 2E11 cells 24 hours after transfection with a vector expressing CoAA-GFP. The HIV-1 transcription site was detected by FISH using a probe corresponding to the MS2 sequence coupled to cy3 (MS2-cy3). Two types of nuclear foci were bleached to follow fluorescence recovery (ordinate): foci positive for GFP and negative for cy3 (corresponding to paraspeckles, PS, in white) and foci positive for GFP and positive for cy3 (corresponding to HIV-1 transcription start site, TSS, in dark).

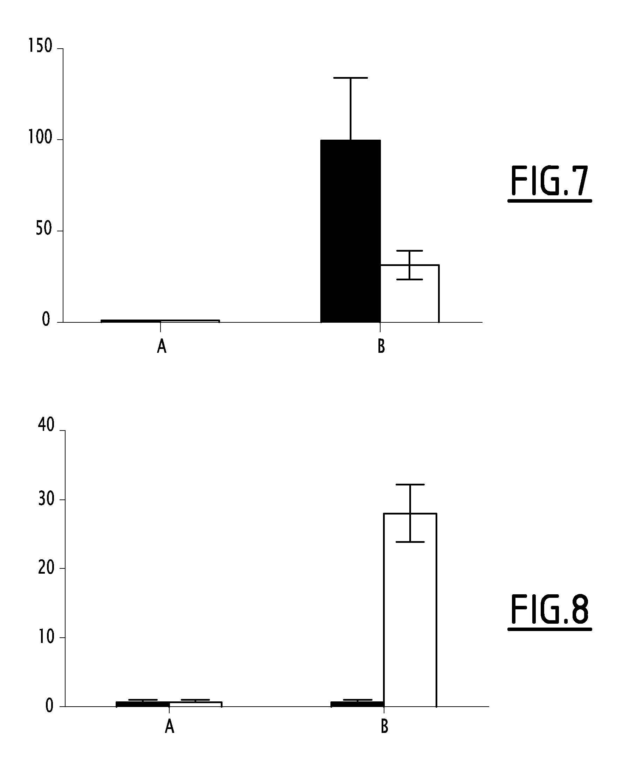

Figure 7. RT-qPCR analyses of spliced HIV-1 reporter RNAs performed in the 2E11 HIV-1 reporter cells 24 hours after transfection with a CoAA-GFP-expressing vector (white bars). In B, cells were treated with a combination of PMA+ionomycin for 15 hours. Results were normalized to that obtained with the control GFP-expressing vector (black bars) in the absence of treatment.
Figure 8: ChIP experiments directed against the HDAC3 protein and performed in 2E11 cells 48 hours after transfection with a CoAA-GFP-expressing vector (white bars). Results are indicated as the calculation of fold enrichment over the no Ab control obtained with the control GFP-expressing vector (black bars). A: no antibody - B: anti-HDAC3 antibody.

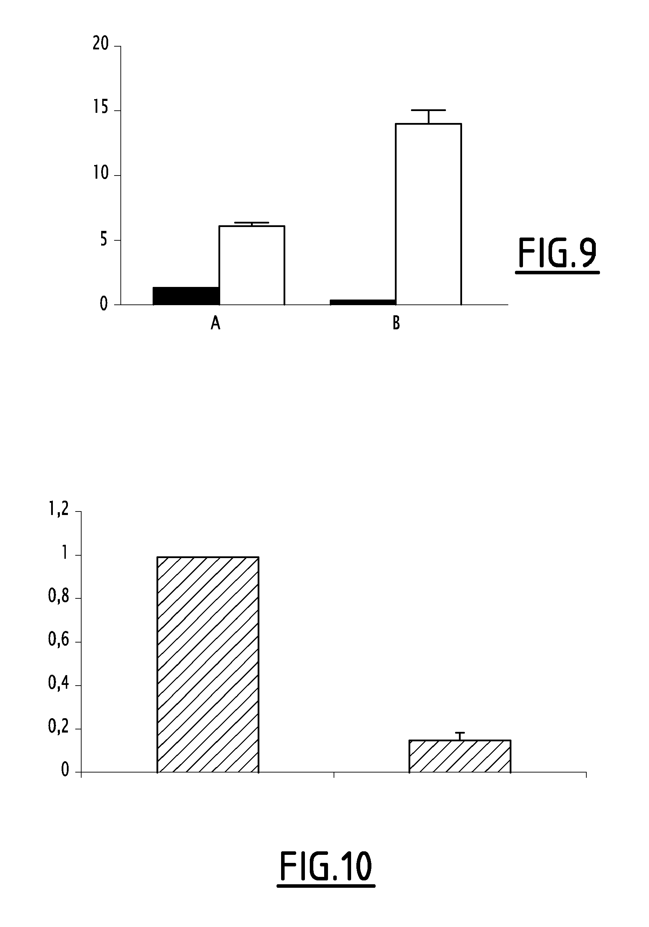

Figure 9: RT-qPCR analyses of spliced HIV-1 reporter RNAs performed in the 2E11 HIV-1 reporter cells 48 hours after transfection with a CoAA-GFP-expressing vector (B) or a GFP-encoding vector used as control (A) and siRNAs directed against HDAC3 (white bars) or control siRNAs (black bars). Results were normalized with GAPDH mRNA and to the results obtained with the control siRNAs in the absence of CoAA (fold change=1). Ordinate: HIV spliced RNA fold change.
Figure 10: The 2E11 cells were transfected with control (A) or anti-CoAA siRNAs (B). RT-qPCR analyses were performed 24 hours post-transfection to evaluate the amount of CoAA mRNA (ordinate: CoAA mRNA fold change). Results were normalized with GAPDH mRNA and to the results obtained with control siRNAs (fold change = 1). These experiments indicated that anti-CoAA siRNAs decreased the amount of CoAA mRNA by 6.7 fold.

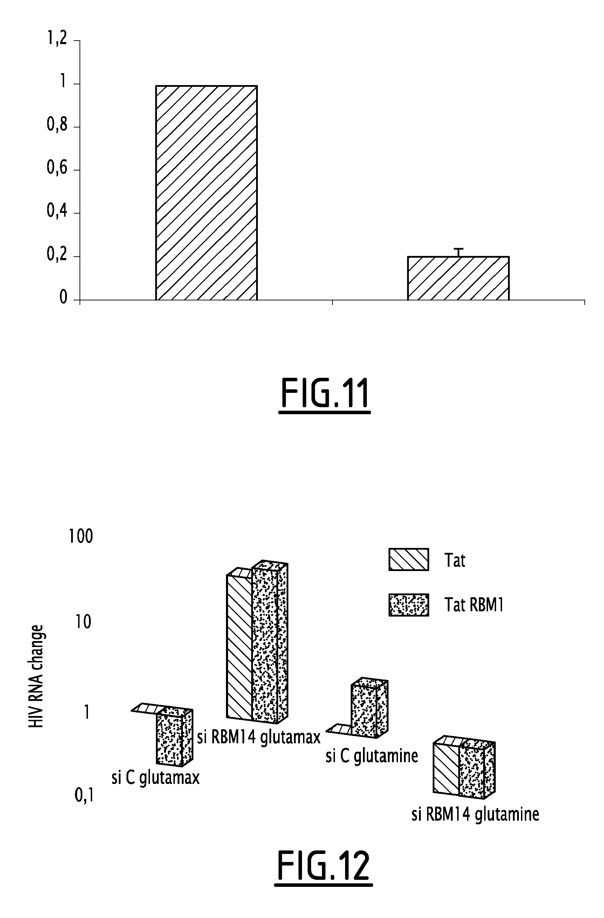

Figure 11: The 2E11 cells were transfected with control (A) or anti-HDAC3 siRNAs (B). RT-qPCR analyses were performed 24 hours post-transfection to evaluate the amount of HDAC3 mRNA (ordinate: CoAA mRNA fold change). Results were normalized with GAPDH mRNA and to the results obtained with control siRNAs (fold change = 1). These experiments indicated that anti-HDAC3 siRNAs decreased the amount of HDAC3 mRNA by 5.3 fold.
Figure 12: 2E11 cells were cultured in two different culture media (DMEM Invitrogen Glutamax or DMEM Invitrogen Glutamine). Cells were then transfected with vectors overexpressing Tat transactivator and CoAA or with a control vector, in combination with control siRNA or siRNA targeting CoAA. HIV RNA fold change was measured.

### Experimental section

### Material and Methods

### Cells and viruses

293T and U2OS cells were maintained in DMEM (Gibco-BRL) supplemented with 2 mM L-glutamine, 100 µg/ml penicillin, 50 µg/ml streptomycin and 10% fetal calf serum and transfected with Lipofectamine 2000 (Invitrogen). Jurkat and Jurkat-CCR5 cells were maintained in RPMI (Gibco-BRL) supplemented with 2 mM L-glutamine, 100 µg/ml penicillin, 50 µg/ml streptomycin and 10% fetal calf serum and transfected using the Amaxa Cell line Nucleofector kit V (Lonza). Cell culture was realized using a Z1 Coulter Particle counter (Beckman Coulter). The Jurkat-CCR5 (#ARP094) were obtained from the Programme EVA Centre for AIDS Reagents, NIBSC, UK, supported by the EC FP6/7 Europrise Network of Excellence, AVIP and NGIN consortia and the Bill and Melinda Gates GHRC-CAVD Project. The prototypes used for infections were HIV-1NL4.3 (X4 tropic), HIV-1NLAD8 (R5 tropic) and HIV-2ROD (X4 tropic). Infections were assessed by RT-PCR. For RT-PCR and semi-nested RT-PCR, the following primers were used: CCAGCNCACAAAGGNATAGGAGG (sense) (SEQ ID NO: 3), N being A, T, G or C, CCCCTATTCCTCCCCTTCTTTTAAAA (antisense) (SEQ ID NO: 4), TGGCARATGGATTGYACACA (sense, semi-nested) (SEQ ID NO: 5). TCID50 determination was performed as previously described (Bjorling et al., Virology,1993, 193:528-530). In brief, frozen supernatants from HIV-1/2-infected PBMC cultures were thawed and serially diluted in 5-fold steps and used to infect 105 PHA-P-stimulated PBMC in five replicate wells in 96-well microtiter plates. Cell culture supernatants were removed and replaced with fresh medium on days 1, 4, and 8 postinfection (p.i.) and the cultures were terminated on day 14 p.i. Supernatants from day 14 p.i. were inactivated with Triton X-100 at a final concentration of 0.5% and stored at -20°C until ELISA detection of HIV-1 and HIV-2 antigens. TCID50 values were obtained using Reed-Muench calculations.

For live cell experiments, U2OS cells were plated on glass, transiently transfected with Lipofectamine with vectors expressing Tat and MS2-GFP, and analyzed 24 h later at 37°C in a nonfluorescent media (Fusco et al., Curr Biol., 2003, 13:161-167). Plasmids expressing Tat and MS2-GFP are described in (Boireau, J Cell Biol., 2007, 179:291-304).

### Plasmids and siRNAs

Pre-miRNAs and anti-miRNAs were purchased from Ambion. The siRNAs (SEQ ID NO:1) directed against CoAA target the following sequence: CCCTTGCCTCCTATGGTAA (CoAA). The siRNAs directed against HDAC3 correspond to a mix of 3 distinct siRNAs:
- CCAAGAGUCUUAAUGCCUU (SEQ ID NO: 6),
- GGCACCCAAUGAGUUCUAU (SEQ ID NO: 7),
- CAUUCAGGAUGGCAUACAA (SEQ ID NO: 8).

Control siRNA corresponds to CCAUGAGCAAGAUGUCCUU (SEQ ID NO: 9). Efficiency of RNAi was controlled by RT-qPCR (Figures 10 and 11).

To construct the CoAA-GFP plasmid, the CoAA cDNA was extracted from the CCSB human ORFeome Collection (version 5.1 -http://horfdb.dfci.harvard.edu/hv5/ index.php?page=orfsearch) and cloned into the pEGFPN1Rfc vector using the Gateway technology.

### Microarrays

Total RNAs were extracted using Tri-reagent (Sigma-Aldrich). RNA sample integrity was assessed using RNA 6000 Nano Assay (based on http://www.icmb.utexas.edu/core/DNA/Information_Sheets/Bioanalyzer/RNA_6000_Nano _Assay.pdf) and Bioanalyzer 2100 (Agilent technologies). High quality RNAs with a ribosomal ratio greater than 1.9 and no evidence of degradation were used in this study. The miRNA microarrays were purchased from LC Sciences (Houston, TX). Experimental conditions and miRNA microarray data analyzes were carried out by the company as described on their website (www.Icsciences.com). The arrays used detect miRNA transcripts listed in Sanger miRBase Release 11.0. Only miRNAs with a p-value < 0.01 were considered as differentially expressed. For cDNA microarrays, two-color microarray-based gene expression analysis was performed using total RNAs and Quick Amp labeling protocol, version 5.7 (March 2008) from Agilent Technologies. Whole human genome microarrays (4x44K, Agilent) were used for hybridization. Microarray images were quantified using the GenePix Pro 6.1 software (Molecular Devices, Sunnyvale, CA). GenePix flagging, able to filter out low-quality spots, was optimized to reduce the number of false negative and false positive spots in the analysis. Information was imported from GenePix Results (GPR) files, processed and further analyzed by R/Bioconductor tools. Spatial effects were removed using two-dimensional approximation of zero log-ratio level by radial basis functions. Dye-effect was removed by a Lowess normalization with the smoothing parameter f=0.67. Next, between-array normalization was performed by median-based centring and scaling of log-ratio distributions. The quality of microarrays was controlled by distributions of log-ratio values of the good-flagged spots, total number of good-flagged spots per array, average correlation with other arrays and spatial homogeneity. Replicate spots were additionally filtered and summarized before the statistical analysis: only spots with intensities significantly higher than the background level were considered. For each condition at least two hybridizations including a dye swap were performed. To find genes with statistically significant regulation in each class (HIV-1 NL4.3 vs. Non Infected cells (NI), HIV-2ROD vs. NI, HIV-1 NLAD8 vs. NI), the empirical Bayes method (Smyth, Stat Appl Genet Mol Biol., 2004, 3:Article 3), implemented in R/Bioconductor's limma package, was used. Genes with adjusted p-value lower than 0.01 were considered as significantly regulated. cDNA microarray data are available in the ArrayExpress database (www.ebi.ac.uk/arrayexpress) under accession number E-MEXP-3139.

### Quantitative RT-PCR

Total RNAs were extracted using Trizol (Invitrogen). RTs were realized using oligodT(N). Primers used for qPCR were as follows: CoAA (sense atcgagtgtgacgtggtgaa (SEQ ID NO: 10), antisense cttctttgccgttgagctg (SEQ ID NO: 11)), CoAM (sense caaagaagtgaagggcaagc (SEQ ID NO: 12), antisense tcagagagctcggctaaacg (SEQ ID NO: 13)) (sense, antisense), spliced HIV-1 RNAs (sense agtctctcaagcggtggt (SEQ ID NO: 14), antisense gactcatcaagtttctctatcaaa (SEQ ID NO: 15)), unspliced HIV-1 RNAs (sense ctgaagcgcgcacggcaa (SEQ ID NO: 16), antisense gacgctctcgcacccatctc (SEQ ID NO: 17)), HDAC3 (sense tgcattgtgctccagtgtg (SEQ ID NO: 18), antisense cttgacatattcaacgcattcc (SEQ ID NO: 19)) and GAPDH (sense gaaggtgaaggtcggagt (SEQ ID NO: 20), antisense gaagatggtgatgggatttc (SEQ ID NO: 21)). The RT-qPCRs were normalized with the level of GAPDH mRNA using the ΔΔCt calculation. Indicated results are means of at least three independent experiments.

### Chromatin Immunoprecipitation

ChIP experiments were performed using a standard protocol (Saumet et al., 2012). The cross-linked chromatin was sonicated and immunoprecipitated overnight at 4°C by using antibodies against GFP (Chromotek) and HDAC3 (Abcam) (4 µg). Immunoprecipitated DNA was used as template for qPCR using the following sets of primers: irrelevant promoter used as negative control (sense gtcgggttccttcaaactca (SEQ ID NO: 22), antisense gctcctgctggcttcttg (SEQ ID NO: 23)), HIV-1 LTR (sense cagagtcacacaacagacg (SEQ ID NO: 24), antisense taactagggaacccactgc (SEQ ID NO: 25)). Fold enrichment over the negative control (no antibody) was calculated using the following formula: 2^{^(Ct no Ab - Ct IP)}. In Figure 5, results are indicated as percentage of input to allow comparison. Indicated results are means of at least three independent experiments.

### Luciferase assays

The psiCHECK-2 Vector (Promega) contains a reporter gene, firefly luciferase, which allows normalization of the Renilla luciferase expression. These vectors were transfected in 293T cells using lipofectamine. Forty-eight hours post-transfection, expression of the renilla and the firefly luciferases were measured with the Dual-Luciferase Reporter Assay System (Promega). Results are the mean of at least 3 independent experiments

### Enzyme-linked immunosorbent assay

The INNOTEST® HIV Antigen mAb ELISA kit was purchased from Innogenetics and experiments were performed according to manufacturer's instructions.

### In situ hybridization

In situ hybridization was performed as previously described (Fusco et al., Curr Biol., 2003, 13:161-167). The formamide concentration was 10% in the hybridization and washing mixture. The sequences of the MS2 probes were as follow (X stands for amino-allyl-T): 5'-AXAGTATTCCCGGGXTCATTAGATCCXAAGGTACCTAATTGCXA (SEQ ID NO: 26).

### FRAP experiments

For live cell imaging, cells were maintained at 37°C in appropriate medium (Fusco et al., Curr Biol., 2003, 13:161-167). A confocal microscope was used (Meta LSM510; Carl Zeiss Microlmaging, Inc.) with a 100x NA 1.4 objective. CoAA-GFP at transcription sites or at paraspeckles was bleached at 488 nm in a circle of 1.5-µm diameter at full laser power and for one passage (bleaching time of 100 ms). Recoveries were measured at a high frame rate (one image for ≤160 ms) and for a short time (up to 10 s) using ≤1% of the power of the 488-nm laser line. Images were analyzed as previously described (Boireau, J Cell Biol., 2007, 179:291-304) by recording the fluorescence of the bleached region. Background was removed, intensities at each time point were corrected for bleaching by dividing them by the total cell fluorescence, and these values were finally normalized by dividing them with the fluorescent intensity before the bleach. Postbleach values were additionally set to zero to facilitate comparison of the curves.

### Modulation of CoAA activity

2E11 cells were cultured in two different culture media (DMEM Invitrogen Glutamax or DMEM Invitrogen Glutamine). Cells were then transfected with vectors overexpressing Tat transactivator and CoAA or with a control vector, in combination with control siRNA or siRNA targeting CoAA. HIV RNA fold change was measured. DMEM Invitrogen Glutamax and Glutamine media have the same composition, except that Glutamax medium comprises L-alanyl-glutamine and Glutamine medium comprises Glutamine.

### Results

### Reconstituting regulation networks in HIV-infected Jurkat T-cells

In order to unveil the functional consequences of miRNA modulations observed with X4 viruses, the transcriptomes of Jurkat-CCR5 T-cells infected for 3 days with HIV-1 NL4.3, HIV-2ROD, HIV-1NLAD8 were profiled using microarrays. For each virus strain, transcripts differentially expressed in infected cells as compared to non-infected cells were derived from a significance analysis considering an adjusted p-value of < 1%. TF- and miRNA-based regulation networks were then reconstituted using a web interface that combines different sequence-based prediction tools to link functionally miRNA, TF and their mRNA targets.

First, in order to facilitate analyses, it was considered the simplest cases where TFs act as transcriptional activator and where the TF mRNA can be correlated with its action. Under these hypotheses, the miRNA and mRNA target profiles should be inversely correlated while TFs and target genes (including miRNAs) profiles should be positively correlated. The analysis was then narrowed according to the following rationale: (i) TFs act as transcriptional activator and thus their modulation correlates positively with that of their target mRNA and miRNA and (ii) miRNA decrease the level of their target and thus their modulation should correlate inversely with that of their target mRNA whether they be TF or not. Two types of networks were reconstituted on whether miRNAs were up- or down-regulated: down-regulated TFs → down-regulated miRNAs → up-regulated genes (type 1) and up-regulated TFs→ up-regulated miRNAs → down-regulated genes (type 2). In addition, to limit the complexity of the networks, it was considered the TFs that were modulated in our experiments only when they were listed in the HIV-1 human protein interaction database or present in the published RNAi screens identifying host factors required for HIV replication. As mentioned above, other networks are possible and can be considered. Though numerous interactions between down-regulated miRNAs and up-regulated genes were identified, our interface failed to predict interactions between down-regulated genes (TFs) and down-regulated miRNAs (type 1 network). On the other hand, a type 2 network was identified implicating only 1 TF, 3 miRNAs and 3 target genes. Among the 3 target genes identified, MDK had previously been shown to act as an inhibitor of HIV-1 replication. Interestingly, RBM14 and CNNM4 had no described function in HIV replication. RBM14 (also known as PSP2) encodes a transcriptional and an RNA splicing modulator. Alternatively spliced transcript variants encoding different isoforms have been described for RBM14 (provided by RefSeq), in particular CoAA and CoAM, which harbor opposing effects on transcription (Iwasaki et al., J Biol Chem. , 2001, 276:33375-33383). Therefore the two RBM14 gene products are hereafter distinguished by using the names CoAA and CoAM. On the other hand, CNNM4 (also known as ACDP4) is supposed to play a role in metal ion transport and mutations in this gene are associated with Jalili syndrome.

### The CoAA protein silences HIV-1 transcription

Because CoAM antagonizes the action of CoAA, it was first determined which product of the RBM14 gene was modulated by HIV. Using specific RT-qPCRs, it was confirmed that the expression of CoAA was down-regulated as early as 4 hours post-infection (Figure 1). On the other hand, CoAM was below detection limit of RT-qPCR in Jurkat T-cells. Hence, the conclusion was that CoAA, rather than CoAM, was the RBM14 gene product potentially implicated in HIV replication. Second, Jurkat T-cells lines were transfected with a vector encoding CoAA-GFP (or a control GFP-expressing vector) and further infected the cells with HIV-1 NL4.3 or HIV-2ROD. The production of the p24 capsid protein was measured 48 hours post-infection (Figure 2). The results obtained with the cells transfected with the GFP-expressing vector were used for normalization (Figure 2). It was observed that forced expression of CoAA decreased the production of the core protein of both HIV-1NL4.3 and HIV-2ROD. Moreover, it was observed that forced expression of CoAA drastically diminished the transcription of both spliced and unspliced HIV-1 RNAs (Figure 3). The 2E11 cellular model of HIV transcription, which consists of HIV-1 reporters containing 24 MS2 repeats stably integrated in tandem in the genome of U2OS cells was also used. Using this model, RT-qPCRs were performed against spliced and unspliced HIV-1 reporter RNAs overexpressing CoAA or inhibiting its expression with specific siRNAs (Figures 10 and 11). It was observed that overexpression of RBM14 resulted in repression of HIV-1 transcription (Figure 4, approximately 4 fold) and that depletion of endogenous RBM14 increased HIV-1 reporter mRNA levels (Figure 4, approximately 6 fold). Of note, we failed to establish Jurkat-T cells stably expressing anti-CoAA shRNA, in agreement with previous observations.

Together these results suggested that CoAA exerts a negative effect on HIV transcription levels. To confirm the RT-qPCR results, Jurkat T-cells were transfected with the pNL4.3 provirus together with a CoAA-expressing vector (or a GFP-expressing vector as a control). Chromatin Immunoprecipitation (ChIP) experiments directed against GFP were performed 48 hours post-transfection (Figure 5). These experiments clearly indicated that CoAA-GFP was able to bind HIV-1 LTR but not an irrelevant promoter sequence. The 2E11 reporter cell line allows, following through FISH experiments directed against the MS2 repeats, the in vivo production of HIV transcripts at the transcription site. These cells were transfected with a CoAA-GFP-expressing vector and FISH experiments were performed 24 hours post-transfection. It was observed that CoAA-GFP was detected in particular nuclear foci, likely corresponding to paraspeckles (data not shown). CoAA-GFP also co-localized with HIV-1 transcription site, confirming the results obtained with ChIP experiments. FISH experiments were also performed in 2E11 cells transfected with flagged CoAA or flagged CoAM-expressing vector (data not shown). While the results obtained with CoAA-GFP in the case of Flag-CoAA (data not shown) were confirmed, a detection of any co-localization of Flag-CoAM with HIV-1 transcription site failed (data not shown), supporting our initial conclusion that CoAA, as opposed to CoAM (or other RBM14 splicing isoforms), is the RMB14 gene product implicated in HIV replication.

CoAA is found in paraspeckles, which are unique subnuclear structures that are built around the long non-coding RNA NEAT1. Recently, NEAT1 was shown to interfere with HIV replication. It was thus tested whether HIV-1 transcription could occur in these particular nuclear foci. For that purpose, the 2E11 cell line wqs transfected with a PSP1-cherry expressing vector, PSP1 being one of the core paraspeckle protein, as well as with a vector expressing the MS2-GFP fusion protein to detect HIV-1 transcripts. Fluorescence observed 24 hours post-transfection did not indicate co-localization between HIV-1 transcription site and PSP1-cherry (data not shown), arguing against the idea that HIV-1 transcription occurs in paraspeckles. Paradoxically, CoAA is reported as one paraspeckle protein. CoAA might then exhibit both localization and harbor different features when localized in paraspeckles or at the HIV-1 transcription site. To clarify this proposal, the 2E11 cells were transfected with vectors expressing MS2-cherry and CoAA-GFP and Fluorescence Recovery After Photobleaching (FRAP) analyses were performed 24 hours post-transfection. The MS2-cherry allowed the detection of HIV-1 transcription site (Figure 6) and helped distinguish the fraction of CoAA-GFP detected in paraspeckles (in white, Figure 6) from the fraction of CoAA located at the HIV-1 transcription site (in dark, Figure 6). These two types of CoAA foci were bleached and fluorescence recovery was measured in both locations (Figure 6). Repeated FRAP analyses indicated that CoAA was more mobile at the HIV-1 LTR than in paraspeckles (Figure 6). Moreover, they revealed the existence of an immobile fraction of CoAA in paraspeckles (around 20%) that does not exist at HIV-1 transcription site (Figure 6). Together, these results confirmed that CoAA is present at the HIV-1 transcription site, where it exhibits specific features that are not encountered in paraspeckles (presumably because it interacts with different protein partners).

### The CoAA protein controls the recruitment of HDAC3 on HIV-1 LTR

The mode of action of CoAA on HIV transcription was then determined. First, it was tested whether the action of CoAA on HIV transcription required the viral Tat transactivator. In fact, though HIV RNAs are faintly expressed in the 2E11 model in the absence of Tat, it is possible to detect some HIV-1 transcription sites. Using FISH experiments and transfection of CoAA-GFP expressing vector, it was observed that CoAA co-localized with HIV transcription site even in the absence of Tat (data not shown). These results were confirmed using RT-qPCRs. The E211 cells were transfected with a CoAA-GFP expressing vector (or a GFP expressing vector as a control) and further treated (or not) with a combination of PMA and ionomycin to mimic the action of Tat (Figure 7). HIV reporter RNAs were faintly detectable by RT-qPCR in the absence of PMA/ionomycin treatment (A, Figure 7), in accordance with our FISH experiments, but their expression was drastically increased in the presence of treatment (PMA+ionomycin, B, Figure 7). In these settings, forced expression of CoAA-GFP severely compromised the effect of PMA and ionomycin (Figure 7), first, confirming the inhibitory action of CoAA on HIV transcription and, second, demonstrating that CoAA does not require the viral transactivator Tat to limit HIV transcription.

One mechanism able to control HIV transcription is the recruitment of histone deacetylases (HDAC). This mechanism is implicated in HIV latency and inhibitors of HDAC are now envisaged in anti-HIV therapies. First, 2E11 cells were transfected with a CoAA-GFP-expressing vector (or a GFP-expressing vector as a control) and ChIP experiments directed against HDAC3 were performed. The binding of HDAC3 on HIV-1 LTR was further assessed by specific qPCR. As shown in Figure 8, in the absence of CoAA, HDAC3 was barely detected on HIV-1 LTR. In contrast, forced expression of CoAA drastically increased the binding of HDAC3 on HIV-1 LTR (Figure 8). These results suggested that CoAA is implicated in the recruitment of HDAC3 on HIV-1 LTR. 2E11 cells were then transfected with siRNAs directed against HDAC3 (Figures 10 and 11) and a vector encoding the Tat transactivator in the presence or absence of CoAA-GFP and measured the level of HIV-1 spliced RNAs (Figure 9). It was confirmed that, in the presence of control siRNAs and Tat, CoAA decreased the amount of HIV spliced RNAs (~ 4 fold, Figure 9). Anti-HDAC3 siRNAs increased the amount of HIV-1 RNAs (~ 5 fold, Figure 9), indicating that HDAC3 did limit the transcription of HIV-1 LTR in the 2E11 model. Importantly, in the presence of anti-HDAC3 siRNAs, CoAA was not able to repress HIV-1 transcription (Figure 9). HIV-1 transcription was even increased (~ 2 fold, Figure 9, compare white bars in the presence/absence of CoAA). Together, our results showed that CoAA recruits HDAC3 on HIV-1 LTR (Figure 8) and that HDAC3 is required for CoAA-mediated inhibition of HIV transcription (Figure 9).

Similar results were obtained with HDAC1, HDAC2 and HDAC8, after transfection of 2E11 cells with vectors overexpressing RBM14 in association with siRNA control or targeting CoAA, HDAC1, HDAC2 and HDAC8. COAA thus acts as a general recruiter of class I HDAC on HIV promoter.

### Modulation of CoAA activity

As shown in figure 12, L-gutamine and L-alanyl-L-glutamine were found to be modulators of CoAA activity. L-glutamine acts as a pro-transcription modulator, whereas L-alanyl-L-glutamine acts as an anti-transcription modulator.

### Conclusions

A novel repressor of HIV transcription, the Co-Activator Activator CoAA, was identified and its mode of action explained. In particular, CoAA inhibits HIV-1 transcription by the recruitment of class I HDACs on HIV promoter.

Besides, new compounds able to modulate CoAA activity were found: in the presence of L-glutamine, CoAA promotes transcription, whereas in the presence of L-alanyl-L-glutamine, CoAA represses transcription.

Agents which modulate CoAA expression and/or activity can thus be used for the treatment of viral diseases in latent form, such as HIV-1, but also in the treatment of diseases that need a repression of transcription, such as viral diseases in proliferative form and cancer.

## Claims

1. An agent which modulates CoAA expression and/or activity for use in the treatment of a disease selected from the group consisting of viral diseases and cancer.

2. The agent for the use according to claim 1, wherein said agent modulates class I HDAC recruitment by CoAA.

3. The agent for the use according to claim 1 or 2, wherein said disease is a viral disease in latent form and wherein said agent is selected from the group consisting of a CoAA expression inhibitor, a RBM14 splicing modulator that increases splicing towards CoAM protein, an agent which interferes with class I HDAC recruitment by CoAA and their combinations.

4. The agent for the use according to claim 3, wherein said CoAA expression inhibitor is selected from the group consisting of siRNA, shRNA, miRNA, dsRNA, aptamer, antisense nucleic acid, peptide nucleic acid, antibody and their combinations.

5. The agent for the use according to claim 3 or 4, wherein said agent which interferes with class I HDAC recruitment by CoAA is L-glutamine.

6. The agent for the use according to any one of claims 3 to 5, wherein said viral disease in latent form is caused by a virus selected from the group consisting of HIV-1, HTLV-1, Rubella, HBV, a virus of the *Herpesviridae* family, AdV 40 and AdV 41.

7. The agent for the use according to any one of claims 3 to 6, wherein said agent is a CoAA siRNA comprising or consisting of a sequence hybridizing to sequence SEQ ID NO: 1 or SEQ ID NO: 2.

8. The agent for the use according to claim 1 or 2 wherein said disease is a viral disease in proliferative form and/or cancer and wherein said agent is selected from the group consisting of a CoAM expression inhibitor, a RBM14 splicing modulator that increases splicing towards CoAA protein, an agent which promotes class I HDAC recruitment by CoAA and their combinations.

9. The agent for the use according to claim 8, wherein said CoAM expression inhibitor is selected from the group consisting of siRNA, shRNA, miRNA, dsRNA, aptamer, antisense nucleic acid, peptide nucleic acid, antibody and their combinations.

10. The agent for the use according to claim 8 or 9, wherein said agent which promotes class I HDAC recruitment by CoAA is L-alanyl-L-glutamine.

11. The agent for the use according to any one of claims 1 to 10 in combination with at least one antiviral drug and/or at least one anti-cancer drug.

12. A CoAA expression inhibitor selected from the group consisting of a siRNA comprising or consisting of a sequence hybridizing sequence to SEQ ID NO: 1 or SEQ ID NO: 2.

13. Composition comprising at least one agent which modulates CoAA expression and/or activity and at least one drug, wherein said drug is an antiviral drug and/or an anti-cancer drug.
